# EUROPEAN PATENT APPLICATION

(11) **EP 2 169 071 A1**
(43) Date of publication of application: **31.03.2010**
(21) Application number: 08017135.8
(22) Date of filing: 29.09.2008
(51) Int. Cl.: C12N 15/85, A01K 67/027, C12N 9/12, C07K 14/71

(54) **Rodent cancer model for human FGFR4 Arg388 polymorphism**

(71) Applicant: Max-Planck-Gesellschaft zur Förderung der Wissenschaften e.V., 80539 München (DE)
(72) Inventor: Ullrich, Axel, 80331 München (DE); Mayr, Thomas, 81373 München (DE); Streit, Sylvia, 82152 Martinsried (DE); Seitzer, Nina, 81241 München (DE)
(74) Representative: Weiss, Wolfgang

(57) **Abstract**

The present invention provides a rodent animal for studying the molecular mechanisms and physiological processes associated with uncontrolled cell growth, e. g. cancer, and with a modified FGFR4.

## Description

### State of the Art

The fibroblast growth factor receptor (FGFR) signaling system is composed of four receptors (FGFR1-4) and more than 20 ligands and has been implicated in the regulation of various physiological processes including angiogenesis, mitogenesis, differentiation and development (1,2).

In human cancer, the FGFRs are implicated either by overexpression like, pancreatic- or prostate carcinoma (3-5), or by activating mutations leading to abnormal fusion proteins or nucleotide substitutions (6,7). In recent years, it has become clear that beside somatic mutations, germline mutations like single nucleotide polymorphisms (SNP) have an increasingly recognized significance for diseases like cancer but also in the determination of the response to therapeutic agents (8,10).

In the human *FGFR4,* a polymorphic nucleotide change in codon 388 substitutes Glycine (Gly) to Arginine (Arg) in the transmembrane region of the receptor, a hot spot in receptor tyrosine kinases (RTKs) for disease-relevant sequence variations (11). This single substitution in the FGFR4 was shown to be implicated in progression and poor prognosis of various types of human cancer. Here, Bange and colleagues could associate the FGFR4 Arg388 allele with tumor progression in breast and colon cancer patients (11). Similarly, soft tissue sarcoma patients, who carried the FGFR4 Arg388 allele had a poor clinical outcome (10). In melanoma, the Arg-allele is associated with increased tumor thickness, while in head and neck squamous cell carcinoma the Glycine-Arginine substitution results in reduced overall patient survival and advanced tumor stage. Furthermore, a recent study on prostate cancer patients strongly associated the FGFR4 Arg-allele not only with tumor progression but also with prostate cancer initiation. Breast cancer studies correlate the Arg-allele not only with accelerated disease progression but also with higher resistance to adjuvant systemic or chemotherapies in primary breast cancer leading to a significantly shorter disease-free and overall survival (11,16).

The main conclusion of these studies was that the presence of one or two Arg388 alleles in the genome of an individual does not initiate cancer development but predisposes the carrier to a more aggressive form if she or he is affected by the disease. Unfortunately, due to the highly complex and heterogeneous genetic background of the studies was at times marginal and because of difference in patient stratification and statistical evaluation, led to controversies (17,18).

The consequences of genetic modifications of the FGFR4 are described in humans suffering from different cancers (see above), however the molecular and biochemical mechanisms and the physiological processes behind them are not understood. Since an impact of the FGFR4 Arg388 allele on tumor progression is shown in correlative clinical studies, understanding the molecular and biochemical alterations underlying such FGFR4 modifications is fundamental for the prognosis on disease, the development of therapeutic strategies, and further cancer research.

Thus, there is a need for an animal as a model to study the molecular and biochemical effects of the FGFR4 modifications, particularly SNPs, leading to amino acid substitutions, in order to develop novel therapeutic strategies, to identify diagnostic markers and agents useful in disease treatment, and to gain more insight in the onset and progression of cancer but also of further diseases associated with FGFR4 modifications.

### Summary of the Invention

In order to satisfy this need, the present invention provides a rodent animal comprising an endogenous gene encoding a modified FGFR4 protein, wherein the modification is an amino acid substitution in the wild-type FGFR4 of said rodent at the amino acid position corresponding to amino acid position 385 of SEQ ID NO: 1.

In an further aspect the invention relates to a rodent animal comprising an endogenous gene encoding a modified FGFR4 protein, wherein the modification is at least one amino acid substitution at any amino acid position compared to the wild-type FGFR4 protein, preferably an FGFR4 protein according to SEQ ID NO: 1, which modification, if present in at least some or all or essentially all cells of said animal in a heterozygous or homozygous manner, results in a phenotype associated with an alteration in tumor progression and/or formation, e. g. an increased rate of tumor growth and/or metastasis formation compared to a wild-type animal. In a preferred embodiment, said animal additionally expresses in the genome of at least some of its cells a transgene encoding a TGF-α protein.

The invention further relates to modified FGFR4 polypeptide and nucleic acid molecules encoding such modified FGFR4 proteins, as well as primary cells and cell lines derived from an non-human animal as described herein, e. g., a rodent.

The invention further pertains to the use of the animals, primary cells, or cell lines described herein as a model for:
(a) studying the molecular mechanisms of, or physiological processes associated with uncontrolled cell growth, such as cancer and/or metastasis formation, preferably in breast cancer, lung cancer, colorectal cancer, hepatocellular cancer, prostate cancer, melanoma, and/or pancreatic cancer;
(b) identification and/or testing of an agent useful in the prevention, amelioration or treatment of uncontrolled cell growth, such as cancer and/or metastasis formation, preferably in breast cancer, lung cancer, colorectal cancer, hepatocellular cancer, prostate cancer, melanoma, and/or pancreatic cancer;
(c) identification of a protein and/or nucleic diagnostic marker for uncontrolled cell growth, such as cancer and/or metastasis formation, preferably in breast cancer, lung cancer, colorectal cancer, hepatocellular cancer, prostate cancer, melanoma, and/or pancreatic cancer; and/or
(d) studying the molecular mechanisms of, or physiological processes or medical conditions associated with undesirable activity, expression, or production of said modified FGFR4.

### Brief Description of the Figures

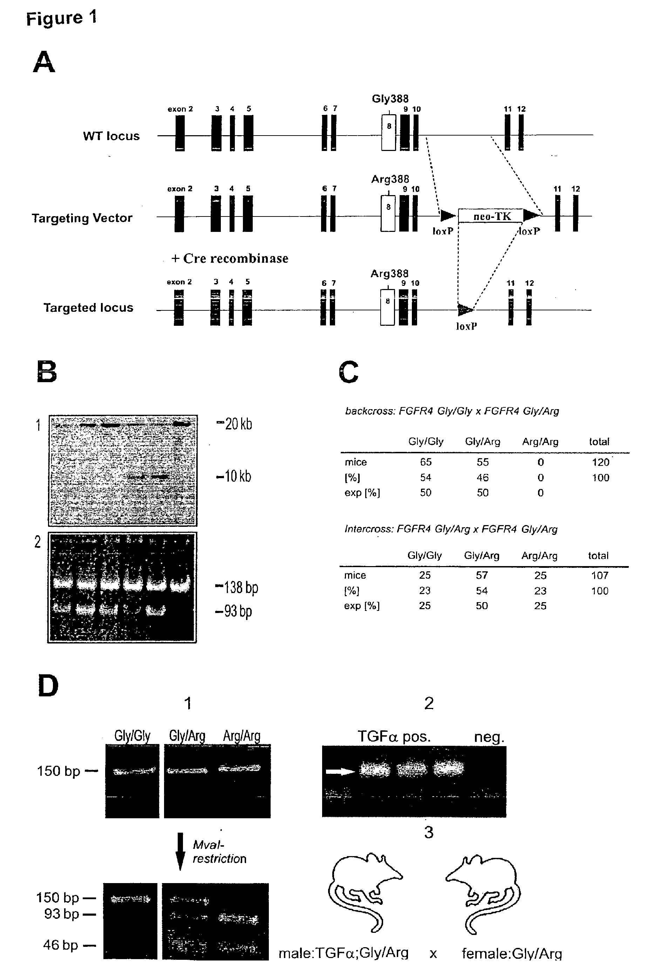

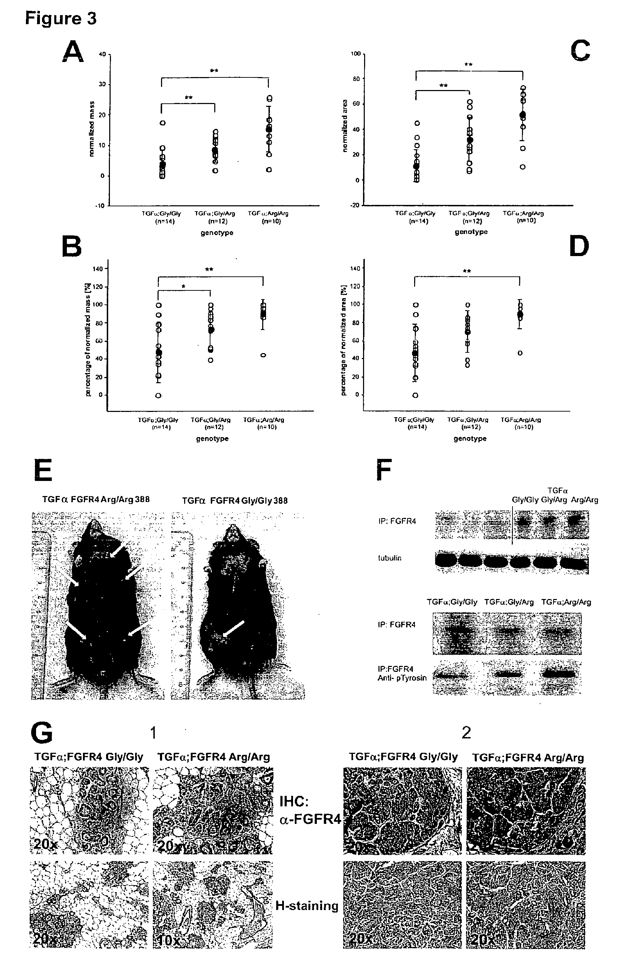

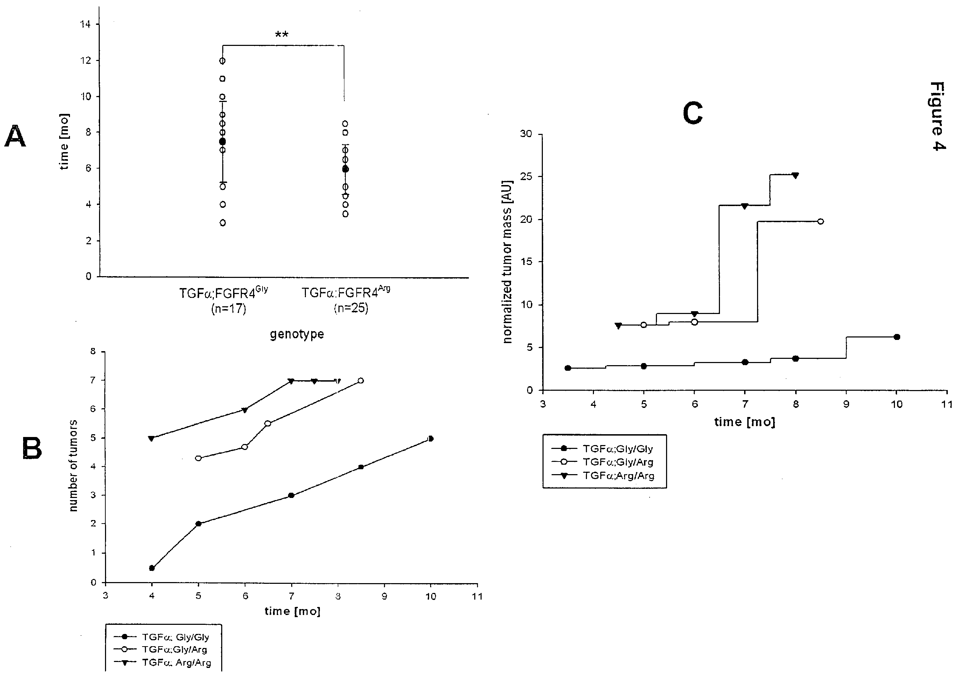

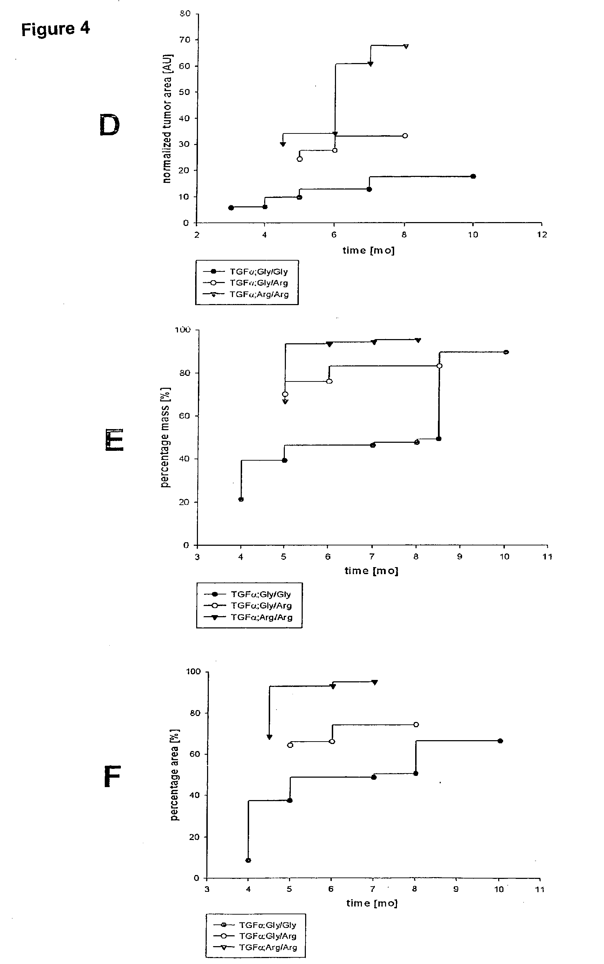

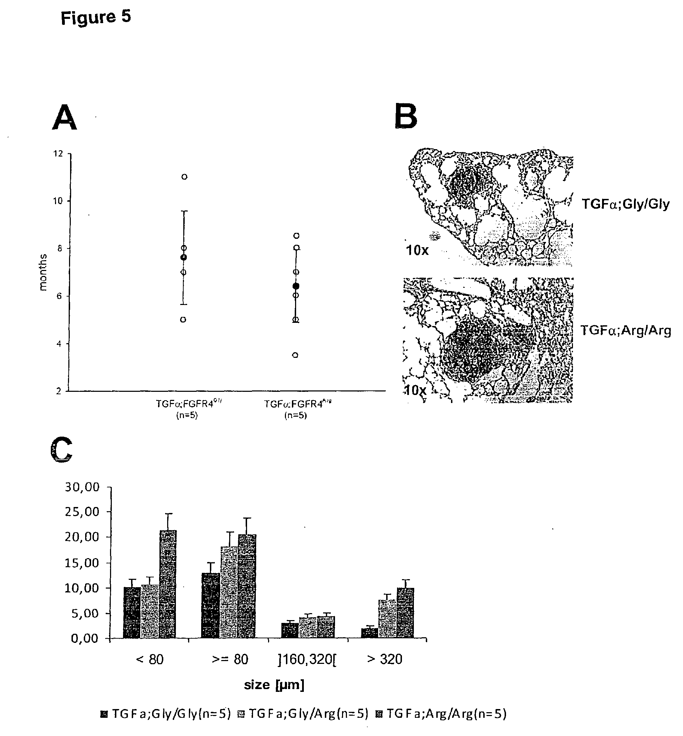

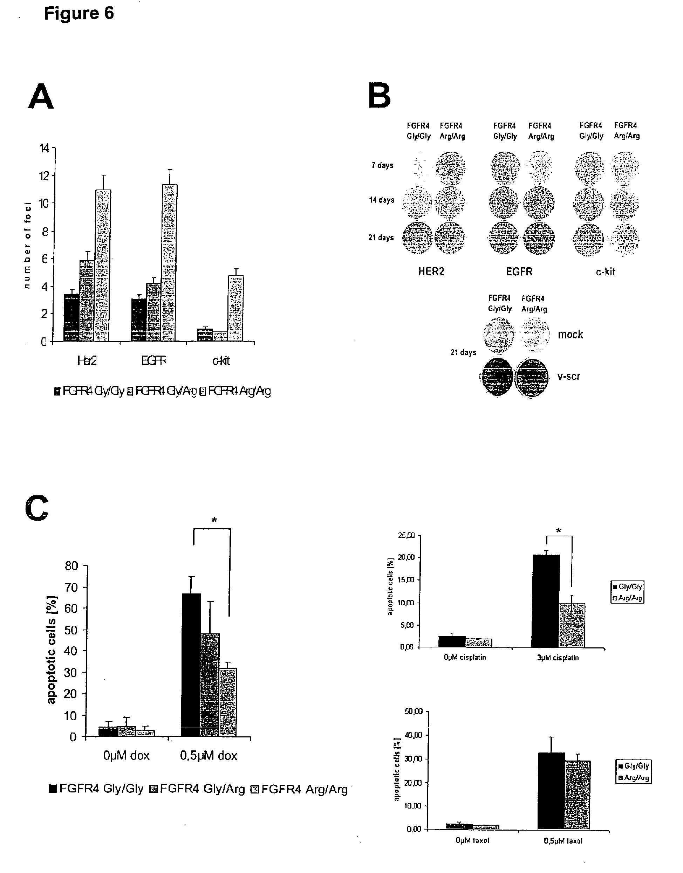

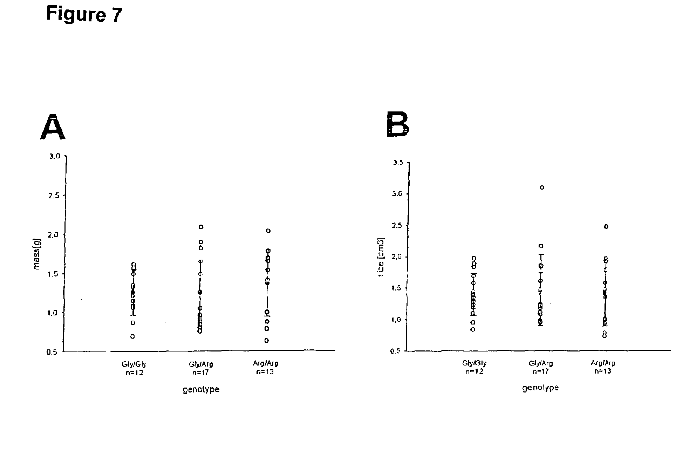

***Figure 1*****:** *Generation* of *the FGFR4 Arg388 KI Mouse*
   (A) *FGFR4* wt locus spanning exon 2 to 12 of murine FGFR4 genomic sequence; *FGFR4 Arg388 KI* gene-targeting construct, exon 8 contains the SNP established via specific mutagenesis, selection-cassette flanked by loxP-sites for Cre-deletion is introduced between exon 10 and 11; Neo: Neomycin-resistance; TK: thymidin-kinase-cassette
   (B) 1) Southern Blot analysis of ES-cell clones after gene targeting; positive clones show a an additional 10 kb detected by 5' external probe 2) genotyping of ES-cell clones via PCR-restriction fragment length polymorphism (RFLP), positive clones contain an additional fragment of 96 bp
   (C) Segregation analysis of *FGFR4 Arg388* KI mice; *FGFR4 Arg388 KI* is inherited with a mendelian ratio in back (1:1) - and intercrosses (1:2:1)
   (D) 1) Genotyping of *FGFR4 Arg388* KI mice; Amplification product was cut by Mval restriction-enzyme to obtain specific banding to distinguish the FGFR4 alleles 2) Conformation of the TGFα transgen; 3) crossing scheme of *FGFR4 Arg388 KI* mice and oncomice transgenic for TGFα; Transgen was only inherited by males to ensure normal lactating of the females.

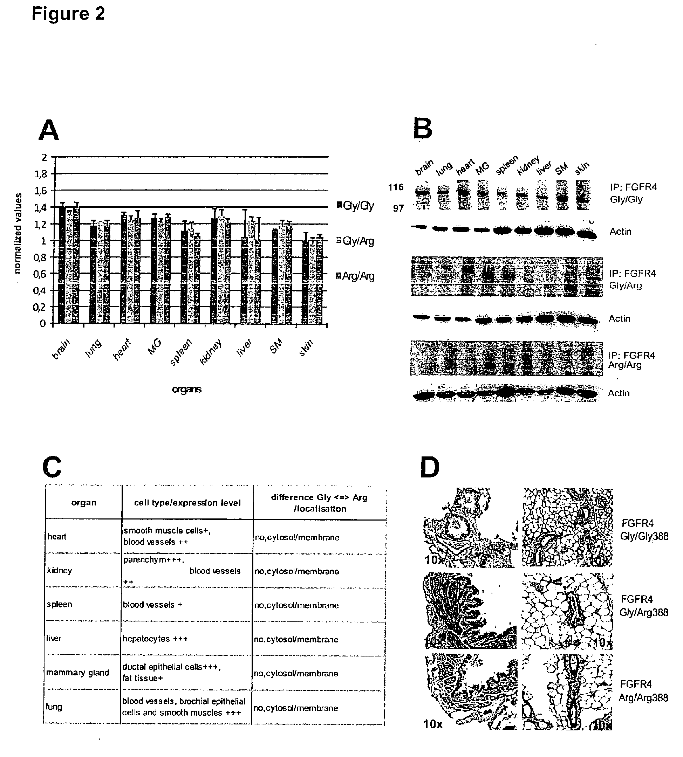

***Figure 2*****:** *Characterisation* of *the FGFR4 Arg388 KI Mouse*
   (A) mRNA expression levels in different tissues of adult *FGFR4 Arg388 KI* mice quantified by LightCycler® analysis; Expression levels are normalised on HPRT-housekeeping gene and plotted as absolute values; FGFR4 is expressed in various tissues; no differences detectable between the different FGFR4 alleles
   (B) Protein-expression levels in different tissues of adult *FGFR4 Arg388 KI* mice analysed by immunoprecipitation and Western Blotting of FGFR4; Actin served as a loading control; no differences detectable between the different FGFR4 alleles
   (C) Table of FGFR4 expression pattern in different tissues of adult *FGFR4 Arg388 KI* mice; FGFR4, immunohistochemically analysed, is expressed in various tissues; no differences detectable between the different FGFR4 alleles
   (D) Lung and mammary gland tissue of adult *FGFR4 Gly*/*Gly, Gly*/*Arg* or *Arg*/*Arg388* KI mice immunohistochemically analysed for FGFR4 expression; no differences detectable between the different FGFR4 alleles
***Figure 3***: *FGFR4 Arg388 enhances Mass and Size of occurring Tumors in the WAP-TGFα*/*EGFR Mouse Mammary Tumor Model*
   In Figure 3 (A-D) every data point represents the values of one mouse. The values were normalised on body weight and plotted against the different investigated genotypes. The median of the values were plotted with asymmetric error bars.
   (A/B) Sum of mass and size of investigated tumors. *FGFR4 Arg388* carrying mice display a significantly increased tumor mass (Gly/Arg-p= 0.03; Arg/Arg-p= 0.002) and tumor size (Gly/Arg-p= 0.01; Arg/Arg-p= 0.0006) comparing to FGFR4 *Gly388.* Hence *FGFR4 Arg388* promotes tumor growth.
   (C/D) Percentage of mass and size of the occurring tumors compared to the whole mammary gland. *FGFR4 Arg388* carrying mice display a significantly increased percentage of tumor mass (Gly/Arg-p= 0.05; Arg/Arg-p= 0.005) and size (Gly/Arg-p= ns; Arg/Arg-p= 0.002) comparing *FGFR4 Gly388.*
   (E) Comparison of FGFR4 Arg/Arg 388 and Gly/Gly 388 mice. White arrows indicate tumors. FGFR Arg/Arg 388 mice show a visible increased tumor mass and number
   (F) Western Blot Analysis of immunoprecipitated FGFR4; FGFR4 is over expressed in WAP-TGFα/EGFR derived tumors compared to non-tumorigenic mammary gland; FGFR4 Arg/Arg displays a higher phosphorylation rate than FGFR4 Gly/Arg or Gly/Gly mice indicating a accelerated activity of the FGFR4 Arg/Arg 388 in WAP-TGFα/EGFR derived tumors
   (G) HE and α-FGFR4 staining of WAP-TGFα/EGFR derived hyperplasic mammary glands and tumors; no obvious pathohistological changes in tumors derived from WAP-TGFα/EGFR mice carrying different FGFR4 alleles were found 1) H and α-FGFR4 staining of WAP-TGFα/EGFR derived hyperplasic mammary glands FGFR4 Arg/Arg is over expressed in hyperplasic mammary glands compared to FGFR4 Gly/Gly expression 2) 1) H and α-FGFR4 staining of WAP-TGFα/EGFR derived tumors; FGFR4 is over expressed and displays no differences between the different alleles
***Figure 4*****:** *FGFR4 Arg388 promotes Tumor Progression in the WAP-TGF*α /*EGFR Mouse Mammary Tumor Model over time*
   In Figure 4 (B-F) every time point indicates the value- median of at least three analysed mice. For every genotype these medians were plotted against time.
   A) Time point of visible tumor incidence in *FGFR4^{Gly388} and FGFR4^{Arg388}; Arg388* carrying mice display a significantly earlier tumor incidence (p= 0.001); the median of the values were plotted with asymmetric error bars. Hence the *FGFR4 Arg388* prematures visible tumor incidence
   (B-D) *FGFR4 Arg388* carrying mice establish a higher number, mass and size of tumors and show a faster progression over time.
   (E-F) *FGFR4 Arg388* carrying mice establish a higher percentage of tumor mass and size and show a faster progression over time
***Figure 5*****:** *FGFR4 Arg388 promotes Cancer Cell Metastasis in the WAP-TGF*α/*EGFR Mouse Mammary Tumor Model*
   (A) Time point of cancer cell metastasis incidence of investigated lungs; Arg388 carrying mice show a earlier occurrence of metastasis (p=ns)
   (B) HE-staining of lung metastases in *FGFR4 GlylGly, GlylArg* and *ArglArg388 KI* mice transgenic for *WAP-TGF*α*;* black arrows indicate metastases; no obvious pathohistological changes were found induced by the different *FGFR4* genotypes
   (C) Analysis of occurred metastases; for every genotype size is plotted against number of metastases; FGFR4 Arg/Arg shows a accelerated number of metastases in every calculated size
***Figure 6*****:** *FGFR4 Arg388 promotes Cellular Transformation and facilitates cellular Survival in Mouse Embryo Fibroblasts (MEFs)*
   (A) Focus Formation Assay in MEFs; *FGFR4 Arg*/*Arg388* carrying MEFs demonstrate a higher number of foci in every used oncogene
   (B) Focus Formation Assay in MEFs; Growing of Foci was determined at different time points; *FGFR4 Arg*/*Arg388* MEFs show an earlier time point of transformation and a higher progression over time
   (C) Apoptosis in MEFs; MEFs were treated with 0.5 µM doxorubicin (dox) for 48 h; apoptosis was measured via FACS Analysis; *FGFR4 Arg*/*Arg388* MEFs display a significantly (p= 0.03) reduced number of apoptotic cells compared to *FGFR Gly*/*Gly* 388 cells; Similarly, Arg388 carrying MEFs display a significantly (p= 0.03) reduced number of apoptotic cells compared to *FGFR Gly*/*Gly* 388 cells after 48 hrs of cisplatin treatment. Contrarily, treatment with taxol, a microtubules-interacting drug, causes no differences in apoptotic response after 48hrs in FGFR4 Gly/Gly 388 MEFs compared to Arg/Arg388 MEFs. Hence FGFR4 Arg/Arg388 seems to facilitate cellular survival in response to DNA damaging drugs
***Figure 7*****:** *FGFR4 Arg388 and Gly388 Mice display identical Mammary Gland-Metrics*
   In Figure 7 (A/B) every data point represents the value of one mouse plotted against the different investigated genotypes. The median of the values were plotted with asymmetric error bars.
   (A) FGFR4 Gly/Gly388, Gly/Arg388 and Arg/Arg388 mice display an identical mass of mammary glands
   (B) FGFR4 Gly/Gly388 Gly/Arg388 and Arg/Arg388 mice display an identical size of mammary glands
***Figure 8*****:** *FGFR4 Arg388 decreases the Time Point of Tumor Incidence in the WAP-TGF*α/*EGFR Model in the FVB Background*
   In Figure 8A every data point represents the value of one mouse plotted against the different investigated genotypes. The median of the values were plotted with asymmetric error bars.
   (A) Tumor onset in FGFR4^{Gly} and FGFR4^{Arg} mice transgenic for WAP-TGFα/EGFR; Arg388 carrying mice transgenic for WAP-TGFα/EGFR display a decreased time point of tumor onset in the FVB background
**Figure 9****:** *Normal Proliferation, Life Span and Migration in FGFR4 Arg388 MEFs*
   (A) FGFR4 Arg388 shows no altered activity in MEFs; FGFR4 was immunoprecipitated and the amount of active receptor was analysed by a p-Tyr antibody; there was no differences detectable between the different FGFR4 alleles;
   (B) FGFR4 Arg388 MEFs display no altered proliferation or a prolonged life span; MEFs were subcultured till senescence occurred, population doubling rate were calculated and plotted against time ; 2) Apparently senescence MEFs were stained for β-galactosidase expression and the amount of senescent cells were calculated microscopically; there was no differences detectable between the different FGFR4 alleles;
   (C) FGFR4 Arg388 MEFs display no altered migration; MEFs were analysed in the Boyden Chamber for migration for 16 h; there was no differences detectable between the different FGFR4 alleles

### Detailed Description of the Invention

The present invention provides a non-human animal, preferably a rodent animal comprising an endogenous gene encoding a modified FGFR4 protein, wherein the modification is an amino acid substitution in the wild-type FGFR4 of said non-human rodent at the amino acid position corresponding to amino acid position 385 of SEQ ID NO: 1. Alternatively, the modification may be a deletion of at least the amino acid at position 385 of SEQ ID NO: 1 or any corresponding sequence or an insertion of at least one amino acid at position 385 of SEQ ID NO: 1 or any corresponding sequence.

The term "corresponding to" as defined herein refers to the amino acid position of FGFR4 orthologues, isoforms, mature forms, or variants as described herein that defines the position 385 of SEQ ID NO: 1 in those sequences. It is obvious to the skilled person that in this context the gene of the modified FGFR4 reflects the modification in the mouse FGFR4 protein according to SEQ ID NO : 1 or any other of the herein-mentioned sequences on the amino acid level. Where the sequences of the FGFR4 gene flanking the modification do not encode amino acids identical to those at the corresponding positions in the amino acid sequences of the mouse FGFR4 protein defined above, the skilled artisan will be readily able to align the amino acid sequences encoded by the flanking sequences with the corresponding amino acids of the mouse FGFR4 protein, preferably by using the below-mentioned method of determining amino acid sequence identity, and determine whether a modification in the mouse FGFR4 protein of the kind mentioned above is reflected by the amino acid sequence encoded by said gene. In case of an amino acid substitution or insertion, the modification is preferably reflected by the amino acid sequence encoded by the gene in such a way that an identical amino acid or amino acid sequence is found at the corresponding position of the protein encoded by the allele. In case of an amino acid deletion, the modification is preferably reflected by the amino acid sequence encoded by the gene in such a way that an identical or corresponding amino acid or amino acid sequence is deleted at the corresponding position of the protein encoded by the gene.

For example, the protein mentioned above may be, for example, a mouse wild-type FGFR4 protein, e. g., with the sequence as disclosed in SEQ ID NO: 1. The modification, e. g., the amino acid substitution, then affects the amino acid position 385 of SEQ ID NO: 1 which is a glycine. Alternatively, the modified FGFR4 protein may be any orthologue of the mouse FGFR4 protein protein according to SEQ ID NO: 1 with respect to the animal, e. g. from from a vertebrate, preferably from a mammal, and more preferably from a rodent, e. g., *Mus* (e. g., mice) or *Rattus* (e. g. rat), or from *Oryctologus* (e. g. Rabbit) or *Mesocricetus* (e. g., hamster). In this case, the amino acid substitution may affect the amino acid position that corresponds to the amino acid position 385 in SEQ ID NO: 1. For example, in the rat sequence according to, e. g., SEQ ID NO: 4, the amino acid position 385 of the mouse sequence corresponds to amino acid position 386.

The modified FGFR4 protein of the non-human animals, e. g. a rodent, as described herein may also be a variant of the mouse or rat FGFR4 protein according to SEQ ID NO : 1 and SEQ ID NO: 4, or of said orthologue, allelic variant or otherwise, wherein certain amino acids or partial amino acid sequences have been replaced, added, or deleted.

Preferably, the amino acid position 385 of the wild type FGFR4 sequence according to SEQ ID NO: 1 in the non-human animals, e. g., a rodent, is replaced by an amino acid different from glycine, e. g., an amino acid with different size and/or polarity, i. e., a non-conservative amino acid substitution. Non conservative substitutions are defined as exchanges of an amino acid by another amino acid listed in a different group of the five standard amino acid groups shown below:
1. small aliphatic, nonpolar or slightly polar residues: Ala, Val, Ser, Thr,(Pro), (Gly);
2. negatively charged residues and their amides: Asn, Asp, Glu, Gln;
3. positively charged residues: His, Arg, Lys;
4. large aliphatic, nonpolar residues: Met, Leu, Ile, Val, (Cys);
5. large aromatic residues: Phe, Trp.

Conservative substitutions are defined as exchanges of an amino acid by another amino acid listed within the same group of the five standard amino acid groups shown above. Three residues are parenthesized because of their special role in protein architecture. Gly is the only residue without a side-chain and therefore imparts flexibility to the chain. Pro has an unusual geometry which tightly constrains the chain. Cys can participate in disulfide bonds.

In one embodiment of the invention, the glycine residue at position 385 of the FGFR4 according to SEQ ID NO: 1 in the non-human animal, e. g. a rodent, is replaced by another residue than glycine, preferably by another residue than Ala, Val, Ser, Thr, (Pro) and preferably with an amino acid with a charged side chain, i. e, with positively charged side chain such as a lysine, arginine or histidine, and more preferably arginine.

In a preferred embodiment the non-human animal, e. g., rodent, expresses the amino acid sequences shown in SEQ ID NO: 5 or SEQ ID NO: 6.

The non-human animal of the present invention, e. g. rodent, is not limited to comprise the modification of the glycine residue at position 385 of the FGFR4 according to SEQ ID NO: 1 or SEQ ID NO: 4 or at the corresponding position in other FGFR4 orthologues. Rather the term "modification" of the FGFR4 of the non-human animals, e. g, rodent, as described herein encompasses any modification in the FGFR4 as long as they do result in the phenotype as described herein, e. g, amino acid substitutions, deletions or insertions. Insertional amino acid sequence modifications are those in which one or more amino acid residues are introduced into a predetermined site in the protein although random insertion is also possible with suitable screening of the product. Deletional modifications are characterized by the removal of one or more amino acids from the sequence leading, e. g., to a frame-shift or insertion of a stop codon. Substitutional amino acid modifications are those in which at least one residue in the sequence has been removed and a different residue inserted in its place.

Accordingly, a further aspect of the invention is a non-human animal, e. g, rodent, comprising an endogenous gene encoding a modified FGFR4 protein, wherein the modification is an amino acid modification as described above, e. g., an amino acid substitution at at least one amino acid position compared to the wild-type FGFR4 protein, preferably an FGFR4 protein according to SEQ ID NO: 1, which modification, if present in at least some or all or essentially all cells of said animal in a heterozygous or homozygous manner, results in a phenotype associated with an alteration in tumor progression and/or formation as described herein further below, e. g. an increased rate of tumor growth and/or metastasis formation compared to the wild-type animal. In a preferred embodiment said animal additionally expresses in the genome of at least some of its cells a transgene encoding a TGF-α protein, e. g, in mammary cells by expression under the control of an appropriate promotor, e. g., the WAP-promotor .

Preferably, the modified FGFR4 in the non-human animal of the invention, e. g., rodent, is at least 70%, 75%, 80%, 85%, 90%, 95%, 98%, or 99% identical with the wild-type FGFR4 sequence of the animal, preferably, the vertebrate, more preferably the mammal and most preferably the rodent, e. g., mouse or rat FGFR4 according to SEQ ID NO: 1 or SEQ ID NO: 4. In one embodiment the modified FGFR4 protein is identical to its wild-type protein except for the amino acid substitution corresponding to amino acid position 385 of the FGFR4 according to SEQ ID NO: 1.

The following definitions apply to any reference to nucleic acid or amino acid sequence identity throughout the present specification:
The term "sequence identity" refers to the degree to which two polynucleotide, protein or polypeptide sequences are identical on a residue-by-residue basis over a particular region of comparison.

The phrases "percent amino acid identity" or "% amino acid identity" refer to the percentage of sequence identity found in a comparison of two or more amino acid or nucleic acid sequences. Percent identity can be readily determined electronically, e. g., by using the MEGALIGN program (DNASTAR, Inc., Madison Wis.). The MEGALIGN program can create alignments between two or more sequences according to different methods, one of them being the clustal method. See, e. g., Higgins and Sharp (Higgins and Sharp1988). The clustal algorithm groups sequences into clusters by examining the distances between all pairs. The clusters are aligned pairwise and then in groups. The percentage similarity between two amino acid sequences, e. g., sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no homology between the two amino acid sequences are not included in determining percentage similarity.

Percent identity can also be readly determined electronically, by using the MultAlin software (Corpet 1988).

Another method of determining amino acid identity between two protein sequences for the purposes of the present invention is using the "Blast 2 sequences" (bl2seq) algorithm described by Tatusova et al. (Tatusova and Madden 1999). This method produces an alignment of two given sequences using the "BLAST" engine. On-line access of "blasting two sequences" can be gained via the NCBI server at http://www.ncbi.nlm.nih.gov/blast/bl2seq/bl2.html. The stand-alone executable for blasting two sequences (bl2seq) can be retrieved from the NCBI ftp site (ftp://ftp.ncbi.nih.gov/blast/executables). Preferably, the settings of the program blastp used to determine the number and percentage of identical or similar amino acids between two proteins are the following :
Program: blastp
Matrix: BLOSUM62
Open gap penalty : 11
Extension gap penalty: 1
Gapxdropoff : 50
Expect: 10.0
Word size: 3
Low-complexity filter: on

The comparison of two or more amino acid or nucleic acid sequences to determine sequence identity can be performed between orthologue sequences, preferably between mouse and rat sequences.

Preferably, the wild type residue of the modified FGFR4 protein wherein the modification is at least one amino acid substitution compared to the wild-type FGFR4 protein, preferably an FGFR4 protein according to SEQ ID NO: 1, which modification, if present in at least some or all or essentially all cells of an animal as described herein, e.g., a rodent, in a heterozygous or homozygous manner, results in a phenotype associated with an alteration in tumor progression and/or formation, e.g. an increased rate of tumor growth and/or metastasis formation compared to a wild-type animal is replaced by an amino acid with different size and/or polarity, i. e., a non-conservative amino acid substitution, as defined above. In a preferred embodiment said animal additionally expresses in the genome of at least some of its cells a transgene encoding a TGF-α protein

The term "phenotype" as used herein refers to one or more morphological, physiological, behavioral and/or biochemical traits possessed by a cell or organism that result from the interaction of the genotype and the environment. Thus, the non-human animal of the present invention, e. g. rodent, displays one or more readily observable abnormalities compared to the wild type animal. In a preferred embodiment the animal of the invention shows at least 1, at least 2, at least 3, or at least 4 abnormal phenotypical features selected from any of the above categories.

The term "phenotype associated with an alteration in tumor progression" as referred to throughout the present application may be characterized by an increased rate of tumor growth and/or metastasis formation compared to a wild-type animal. Further characterization that fall under the definition of the this phenotype may be found below in the Examples. A preferred tumor in this respect is a mammary tumor.

The endogenous promoter of the FGFR4 gene or transgene as described above in connection with the non-human animals, e. g. rodent, may be replaced by a heterologous promoter, e. g., a promoter imposing a different tissue specificity of expression upon the gene e. g., the WAP-promotor for mammary cells, villin-promotor for colorectal cells, or the albumin promotor for hepatocytes or a temporally controlled promotor, e. g., a promoter that is inducible by chemical or physical means, e. g., the tet-CRE system.

The term "modified" or "modification" as used herein refers to an alteration compared to the wild type. The term "mutant" or "modified" as used herein in connection with the FGFR4 protein sequences and nucleic acid sequences relating thereto refers to an alteration in the sequence compared to the corresponding wild type FGFR4.

The non-human animals as described herein may be a vertebrate animal, preferably a mammal. In a further preferred embodiment, the non-human animal is a rodent.

In particular, the rodent may be selected from the genus *Mus* (e. g., mice), *Rattus* (e. g. rat), *Oryctologus* (e. g. Rabbit) or *Mesocricetus* ( e. g., hamster). A particular preferred non-human animal is a mouse.

The non-human animal models of the invention as described herein, e. g. a rodent, expresses the endogenous modified FGFR4 protein as described herein or the transgene as described herein in at least some of its cells, e. g., as mosaic animal, such as chimeric animals, or in case the modified FGFR4 protein is expressed by a gene with a heterologous promotor as defined above. The non-human animal model of the invention, e. g. a rodent, may also express the endogenous modified FGFR4 protein as described herein in all of its cells, e. g., by expressing the FGFR4 protein from a nucleic acid encoding said FGFR4 under the control of an ubiquitarily expressed promotor. The modified FGFR4 protein may also be translated from a nucleic acid encoding the nucleic acid encoding said FGFR4 protein under the control of the endogenous FGFR4 promotor. The cells of the non-human animal as described, e. g. rodent, are at least heterozygous with respect to the amino acid modification, e.g., substitution, as described herein. Alternatively, the cells may also be homozygous.

The invention furthermore encompasses non-human animals, e g. a rodent, comprising mature modified FGFR4 proteins, or their vertebrate orthologues being modified as described herein, e. g., the specific orthologues referred to above, which comprise an amino acid or amino acid sequences corresponding to the FGFR4 proteins as defined herein. As used herein, a "mature" form of a polypeptide or protein may arise from a posttranslational modification. Such additional processes include, by way of non-limiting example, proteolytic cleavage, e. g., cleavage of a leader sequence, glycosylation, myristoylation or phosphorylation. In general, a mature polypeptide or protein according to the present invention may result from the operation of one of these processes, or a combination of any of them.

The nucleic acid or gene encoding the amino acid substitution of the invention may be present in germ cells or somatic cells of the non-human vertebrate animal, or both.

The non-human animals as described herein, e. g. a rodent, may in addition to the modification of the FGFR4 as described herein displaying uncontrolled cell growth, preferably cancer and/or metastasis formation.

The term "uncontrolled cell growth" as used in the present invention relates to any state characterized by uncontrolled growth, e. g., cancer. Examples of cancer are breast cancer, lung cancer, colorectal cancer, hepatocellular cancer, prostate cancer, melanoma, and/or pancreatic cancer. The term "uncontrolled cell growth" also comprises uncontrolled division of cells, i. e., growth and/or division beyond the growth and/or division of the same cells in a non-uncontrolled cell growth state. Techniques how to determine uncontrolled cell growth are known by the person skilled in the art, e. g., visual inspection of the cells (histology).

Uncontrolled cell growth may be triggered by any method or treatment that is known by the skilled person to lead to uncontrolled growth and/or division of cells, i. e., irradiation, e. g., with UV-light ,or treatment with a cancer-inducing agent, e. g., dimethylhydrazine (DMH), azoxymethane (AOM), N-methyl-N-nitro-N-nitrosoguanidine (MNNG), N-methyl-N-nitrosourea (MNU), ethyl-nitroso-urea (ENU) or 12-0-tetradecanoylphorbol-13-acetate (TPA). Alternatively, it may be triggered by the expression of a transgene comprising an oncogene, i. e., a gene which is deregulated and which deregulation participates in the onset and development of cancer. Examples of such genes are TGF-α, TGF-β, HGF, IGF-I, PyV-mz, erb-B2, RET, Cyclin D1, Cyclin A, myc, p53, ras, Rb, particularly TGF-α, TGF-β, erb-B2, p53, myc, or ras. The transgene may be expressed in the whole organism or in individual cells or tissues, e. g., in mammary cells, lung cells, colorectal cells, hepatocytes, prostate cells, skin cells, or pancreatic cells, particularly β-islets. As described above, expression of the transgene in all or at least some cells may be achieved by the use of appropriate promotors.

In an further aspect of the invention, the non-human animal, e. g. rodent, comprising an endogenous gene encoding a modified FGFR4 protein, wherein the modification is, e. g., an amino acid substitution in the wild-type FGFR4 of said non-human animal at the amino acid position corresponding to amino acid position 385 of SEQ ID NO: 1 as described herein develop a phenotype associated with an alteration in tumor progression and/or formation as described herein.

In another aspect, the non-human animal, e. g. rodent, comprising an endogenous gene encoding a modified FGFR4 protein, wherein the modification is a modification as described herein, e. g., an amino acid substitution at at least one amino acid position compared to the wild-type FGFR4 protein, preferably an FGFR4 protein according to SEQ ID NO:1, which modification, if present in at least some or all or essentially all cells of said animal in a heterozygous or homozygous manner, results in a phenotype associated with an alteration in tumor progression and/or formation as described herein, e.g. an increased rate of tumor growth and/or metastasis formation compared to a wild-type animal develops a phenotype associated with an alteration in tumor progression and/or formation as described herein. In a preferred embodiment said animal additionally expresses in the genome of at least some of its cells a transgene encoding a TGF-α protein.

In an further aspect of the invention, the non-human animal, e. g. rodent, comprising an endogenous gene encoding a modified FGFR4 protein, wherein the modification is an amino acid substitution in the wild-type FGFR4 of said non-human animal, e. g. rodent, at the amino acid position corresponding to amino acid position 385 of SEQ ID NO: 1 as described herein displaying in addition to the modification of the FGFR4 as described herein uncontrolled cell growth as described above and/or metastasis formation develops a phenotype associated with an alteration in tumor progression and/or formation as described herein.

In another aspect, the non-human animal, e. g. rodent, comprising an endogenous gene encoding a modified FGFR4 protein, wherein the modification is a modification as described herein, e. g., an amino acid substitution at at least one amino acid position compared to the wild-type FGFR4 protein, preferably an FGFR4 protein according to SEQ ID NO:1, which modification, if present in at least some or all or essentially all cells of said animal in a heterozygous or homozygous manner, results in a phenotype associated with an alteration in tumor progression as described herein, e. g. an increased rate of tumor growth and/or metastasis formation compared to a wild-type animal, displaying in addition to the modification of the FGFR4 as described herein uncontrolled cell growth as described above and/or metastasis formation develops a phenotype associated with an alteration in tumor progression and/or formation as described herein. In a preferred embodiment, said animal additionally expresses in the genome of at least some of its cells a transgene encoding a TGF-α protein.

As will be apparent from the previous explanations, the non-human animals according to the invention, e. g. rodent, may be produced by any technique known to the person skilled in the art, e. g., by the application of procedures, which result in an animal with a genome that incorporates/integrates exogenous genetic material, e. g., in such a manner as to modify or disrupt the function of the normal FGFR4 gene or protein or in such a manner to express a modified FGFR4 as described above or in such a manner as to integrate additional copies of a gene, e. g., a transgene comprising an oncogene as described herein. These techniques may include but are not limited to micro-injection, electroporation, cell gun, cell fusion, nucleus transfer into anucleated cells, micro-injection into embryos of teratocarcinoma stem cells or functionally equivalent embryonic stem cells. One embodiment of a procedure for generating an animal of this invention is one according to "Material and Methods" further below.

In case of production of a transgenic animal with a transgene that comprises an oncogene as described above, the genetic material encoding the transgene may be micro-injected into the pro-nucleus of a fertilized ovum, a process that is known by the skilled person. The insertion of DNA is, however, a random process. The manipulated fertilized ovum is transferred into the oviduct of a recipient female, or foster mother that has been induced to act as a recipient by mating with a vasectomized male. The resulting offspring of the female is likewise tested to determine which animals carry the transgene.

The present invention further provides for inbred successive lines of animals carrying the nucleic acid encoding the modified FGFR4 protein of the present invention that offer the advantage of providing a virtually homogeneous genetic background. A genetically homogeneous line of animals provides a functionally reproducible model system for conditions or symptoms associated with uncontrolled cell growth, with alterations in tumor progression, and/or metastasis formation.

The animals of the invention can also be used as a source of primary cells, e. g., mouse embryonic feeder cells (MEF), from a variety of tissues, for cell culture experiment, including, but not limited to, the production of immortalized cell lines by any methods known in the art, such as retroviral transformation.

Such primary cells or immortalized cell lines derived from any one of the non-human vertebrate animals described and claimed herein are likewise within the scope of the present invention. Such immortalized cells from these animals may advantageously exhibit desirable properties of both normal and transformed cultured cells, i. e., they will be normal or nearly normal morphologically and physiologically, but can be cultured for long, and perhaps indefinite periods of time. The primary cells or cell lines derived thereof may furthermore be used for the construction of an animal model according to the present invention.

In other embodiments cell lines according to the present invention may be prepared by the insertion of a nucleic acid construct comprising the nucleic acid sequence of the invention or a fragment thereof comprising the codon imparting the above-described phenotype to the animal model of the invention. Suitable cells for the insertion include primary cells harvested from an animal as well as cells, which are members of an immortalized cell line. Recombinant nucleic acid constructs of the invention, described below, may be introduced into the cells by any method known in the art, including but not limited to, transfection, retroviral infection, micro-injection,electroporation, transduction or DEAE-dextran. Cells, which express the recombinant construct, may be identified by, for example, using a second recombinant nucleic acid construct comprising a reporter gene, which is used to produce selective expression. Cells that express the nucleic acid sequence of the invention or a fragment thereof may be identified indirectly by the detection of reporter gene expression.

It will be appreciated that the non-human animals of the invention, e. g. rodents, are useful in various respects in connection with phenotypes relating to an alteration in tumor progression and/or formation; with uncontrolled cell growth; with medical conditions associated with uncontrolled cell growth, e.g. cancer, tumor formation and/or progression; with metastasis formation; with uncontrolled cell growth, and/or uncontrolled cell division.

Accordingly, one aspect of the present invention is the use of the non-human animal, e. g. rodent, primary cells, or cell lines as described herein as a model for studying the molecular mechanisms of, or physiological processes associated with uncontrolled cell growth, such as cancer and/or metastasis formation, preferably in breast cancer, lung cancer, colorectal cancer, hepatocellular cancer, prostate cancer, melanoma, and/or pancreatic cancer. This may be done, e. g., by performing differential proteomics analysis, using techniques including , e. g., 2D-gel electrophoresis, protein chip microarrays, or mass spectrophotometry on tissue displaying uncontrolled cell growth, e. g., cancer, as described herein. This may also be done on nucleic acid level by, e. g., differential display or cDNA microarrays.

A further aspect of the invention is the use of the non-human animal, e. g. rodent, primary cells, or cell lines as described herein as a model for studying the identification and/or testing of an agent useful in the prevention, amelioration or treatment of uncontrolled cell growth, such as cancer and/or metastasis formation, preferably in breast cancer, lung cancer, colorectal cancer, hepatocellular cancer, prostate cancer, melanoma, and/or pancreatic cancer. The agent to be tested can be administered to an animals of the present invention, e .g., a rodent, and any technique known by the skilled person may be used in order to monitor the effect of the agent to be tested. The non-human animal, e. g. rodent) may be exposed to the agent to be tested at different stages of uncontrolled cell growth, e. g., cancer and, e. g., the mass, area and percentage of occurring tumors and/or metastasis formation; the percentage of mass and area of the tumor and/or metastasis formation compared to to the whole tissue in which the tumor or metastases occurrs; the expression profile of FGFR4 within the tumor compared to the same tissue without a tumor; the phosphorylation status of FGFR4 in tumor tissue compared to non-tumor tissue, or Focus Formation Assay may be determined (cf. also Examples below).

Also within the scope of the invention is the use of the non-human animal, e. g. rodent, primary cells, or cell lines as described herein as a model for studying the identification of a protein and/or nucleic diagnostic marker for uncontrolled cell growth, such as cancer and/or metastasis formation, preferably in breast cancer, lung cancer, colorectal cancer, hepatocellular cancer, prostate cancer, melanoma, and/or pancreatic cancer, such as diagnostic markers relating to genes or gene products that play a role in the early phase, the intermediate phase, and/or the late phase of medical conditions associated with an uncontrolled cell growth, such as cancer as described herein, or diagnostic markers for diseases associated with FGFR4 modifications as described herein.

It will be appreciated that such diagnostic markers may relate to the FGFR4 gene or its protein product. However, it will be understood that the non-human animal according to the present invention, e. g. a rodent, can also be used to identify markers relating to other genes or gene products that affect FGFR4 gene or protein expression or function, or the expression or function of which is affected by the FGFR4 protein. Moreover, since the non-human animal of the invention, e. g. a rodent, represents a highly useful model system for studying the pathogenesis of medical conditions associated with uncontrolled cell growth, such as cancer as described herein, it will be appreciated that it may also be used to identify disease-relevant markers relating to genes or gene products that do not directly affect FGFR4 gene or protein expression or activity, or the expression or activity of which is not directly affected by the FGR4 protein. It will be appreciated that the above-mentioned uses represent further aspects of the present invention. This may be done, e. g., by performing differential proteomics analysis, using techniques including , e. g., 2D-gel electrophoresis, protein chip microarrays, or mass spectrophotometry on tissue displaying uncontrolled cell growth, e. g., cancer, as described herein. This may also be done on nucleic acid level by, e. g., differential display or cDNA microarrays.

A further aspect of the invention is the use of the non-human animal, e. g. rodent, primary cells, or cell lines as described herein as a model for studying the molecular mechanisms of, or physiological processes or medical conditions associated with undesirable activity, expression, or production of said modified FGFR4. This may be done, e. g., by performing differential proteomics analysis, using techniques including ,e. g., 2D-gel electrophoresis, protein chip microarrays, or mass spectrophotometry on tissue displaying uncontrolled cell growth, e. g., cancer, as described herein. This may also be done on nucleic acid level by, e. g., differential display or cDNA microarrays.

The term "undesirable activity, expression, or production of said modified FGFR4" as used herein refers to any undesirable activity, expression, or production of the protein and/or gene encoding said modified FGFR4. The undesirable activity, expression, or production may relate to any aberrant activity, expression or production, i.e., activity, expression or production beyond the normal activity, expression or production of FGFR4 as well as any activity, expression or production that is below the normal activity, expression or production of FGFR4.

It will also be appreciated that the non-human animals described herein, e. g., a rodent, as well as the primary cells or cell lines as described herein, will be highly useful as a model system for the screening and identification of binding partners, particularly ligands of the FGFR4 protein, or upstream or downstream genes, or genes or proteins regulated by FGFR4 protein or its gene or protein activity and/or deregulated by expression of a modified FGFR4 or in disorders associated with modified FGFR4 protein. Such agents may be, for example, small molecule drugs, peptides or polypeptide, or nucleic acids.

It will also be appreciated that the non-human animals described herein, e. g., a rodent, as well as the primary cells or cell lines as described herein, will be highly useful for studying whether the amino acid modifications of the FGFR4 in mammary tumors as described herein plays the same or a similar role in other cancer types, e. g., in hepatocelluar cancer, lung cancer, prostate cancer, colorectal cancer, melanoma, or in pancreatic cancer.

The invention further relates to a modified FGFR4 polypeptide and nucleic acid molecules, e. g., a gene, encoding such a modified FGFR4 protein or polypeptide as described herein in particular in connection with the animals.

Accordingly, the present invention also provides amino acid sequences of a modified FGFR4, for example, murine and rat modified FGFR4 amino acid sequences. The wild type murine and rat FGFR4 amino acid sequences are shown in SEQ ID NO: 1 and SEQ ID NO: 4, respectively. A preferred modified version of FGFR4, e. g., the mouse and rat FGFR4, amino acid sequence is one wherein glycine at position 385 or 386 is mutated to a non-glycine amino acid. A more preferred version of the mouse and rat FGFR4 amino acid sequence is one wherein glycine at position 385 or 386 is mutated to a charged amino acid, e. g., a positively charged amino acid, i. e., lysine, arginine, or histidine. A most preferred version of the mouse and rat FGFR4 amino acid sequence is one wherein glycine at position 385 or 386 is mutated to an arginine (SEQ ID NO: 5 or SEQ ID NO: 6).

Another preferred version of FGFR4 is one with a modification, e. g., an amino acid substitution, at at least one amino acid position compared to the wild-type FGFR4 protein, preferably an FGFR4 protein according to SEQ ID NO: 1, which modification, if present at least some or all or essentially all cells of a non-human animal of the invention, e.g. a rodent, in a heterozygous or homozygous manner, results in a phenotype associated with an alteration in tumor progression and/or formation as described herein, e. g., an increased rate of tumor growth and/or metastasis formation compared to a wild-type animal. In a preferred embodiment said animal additionally expresses in the genome of at least some of its cells a transgene encoding a TGF-α protein.

In one embodiment the modified FGFR4 protein and nucleic sequences as described herein are isolated protein or nucleic acid sequences. An "isolated" or "purified" polypeptide or protein, or a biologically active fragment thereof as described herein, is substantially free of cellular material or other contaminating proteins from the cell or tissue source from which the polypeptide or protein is derived, or substantially free from chemical precursors or other chemicals when chemically synthesized. The language "substantially free of cellular material" includes preparations of the FGFR4 protein in which the protein is separated from cellular components of the cells from which the protein is isolated or in which it is recombinantly produced.

Also encompassed by the present invention are fragments of such proteins comprising at least 6, at least 7, at least 8, at least 9, at least 10, at least 15, at least 20, at least 25, at least 30, at least 35, at least 40, at least 50, at least 60, at least 70, at least 80, at least 90, at least 100, at least 150, at least 200, at least 250, at least 300, at least 350, at least 400, at least 450, at least 460, at least 470, at least480, at least 490, at least 500, at least 550, at least 600, at least 650, at least 700, at least 750, a least 790, at least 791, at least 792, at least 793, at least 794, at least 795, at least 796, at least 797, at least 798, at least 799 or at least 800 contiguous amino acids having the amino acid modifications as described herein, e. g., an amino acid substitution in the wild-type FGFR4, e. g., mouse or rat FGFR4, at the amino acid position corresponding to amino acid position 385 of SEQ ID NO: 1 or an an amino acid substitution at at least one amino acid position compared to the wild-type FGFR4 protein, preferably an FGFR4 protein according to SEQ ID NO: 1 or 386 of SEQ ID NO: 4, which modification, if present at least some or all or essentially all cells of a non-human animal of the invention as described herein, e. g. a rodent, in a heterozygous or homozygous manner, results in a phenotype associated with an alteration in tumor progression and/or formation as described herein, e. g., an increased rate of tumor growth and/or metastasis formation compared to a wild-type animal. In a preferred embodiment said animal additionally expresses in the genome of at least some of its cells a transgene encoding a TGF-α protein.

In a preferred embodiment, the protein of the invention represents an orthologue of the mouse FGFR4 protein according to SEQ ID NO: 5, preferably a vertebrate orthologue. Alternatively, it may represent a mammalian orthologue, in particular a rodent selected from the genus *Mus* (e. g., mice), *Rattus* (e. g. rat), *Oryctologus* (e. g. Rabbit) or *Mesocricetus* (e. g., hamster), preferably the rat orthologue according to SEQ ID NO: 6. It may also be a variant of the mouse FGFR4 protein according to SEQ ID NO: 5, respectively, or of said orthologue, preferably said rat orthologue according to SEQ ID NO: 6, allelic variant or otherwise, wherein certain amino acids or partial amino acid sequences have been replaced, added, or deleted.

Again in a preferred embodiment, the modification mentioned above results in a deletion or substitution by another amino acid of at least one an amino acid of said mouse FGFR4 protein according to SEQ ID NO: 1 or corresponding FGFR4. Alternatively, the modification may result in an insertion of additional amino acids not normally present in the amino acid sequence of the mouse FGFR4 protein or corresponding FGFR4 defined above.

The substitution may furthermore be a substitution of an amino acid by another amino acid, which is a conservative amino acid substitution between mouse and rat FGFR4 as described above. Such an amino acid may be a non-naturally occurring or a naturally occurring amino acid.

Preferably, the wild type residue of the modified FGFR4 protein is replaced by an amino acid with different size and/or polarity as defined above.

The invention furthermore encompasses mature modified mouse FGFR4 or rat FGFR4 proteins, or their vertebrate orthologues, e. g., the specific orthologues referred to above, which comprise an amino acid or amino acid sequences corresponding to a modification as defined herein.

The invention also provides modified FGFR4 based chimeric or fusion proteins. As used herein, a "chimeric protein" or "fusion protein" comprises a FGFR4 protein, either wild type or modified in accordance with the present invention, or a fragment of such protein as defined above, linked to a non-FGFR4 polypeptide (i. e., a polypeptide that does not comprise a FGFR4 protein or a fragment thereof), e. g., amino acid sequences that are commonly used to facilitate purification or labeling, e. g., polyhistidine tails (such as hexahistidine segments), FLAG tags, HSV-tags, a beta-galactosidase tags and streptavidin.

The amino acid sequences of the present invention may be made by using peptide synthesis techniques well known in the art, such as solid phase peptide synthesis (see, for example, Fields et al. ,"Principles and Practice of Solid Phase Synthesis" in SYNTHETIC PEPTIDES, A USERS GUIDE, Grant, G. A. , Ed. , W. H. Freeman Co. NY. 1992, Chap. 3 pp. 77- 183; Barlos, K. and Gatos, D. "Convergent Peptide Synthesis"in FMOC SOLID PHASE PEPTIDE SYNTHESIS, Chan, W. C. and White, P. D. Eds. , Oxford University Press, New York, 2000, Chap. 9: pp. 215-228) or by recombinant DNA manipulations and recombinant expression, e. g. , in a host cell. Techniques for making substitution mutations at predetermined sites in DNA having a known sequence are well known and include, for example, M13 mutagenesis.

Manipulation of DNA sequences to produce variant proteins which manifests as substitutional, insertional or deletional variants are conveniently described, for example, in Sambrook et al.

The present invention provides nucleic acid or gene sequences encoding the FGFR4 proteins as described in more detail above and below, for example FGFR4 modified in accordance with the present invention. In a preferred embodiment, this invention provides a nucleic acid sequence encoding a modified mouse and rat FGFR4 protein as described herein. Modified mouse and rat FGFR4 encoding nucleic acids or genes, can be made, for example, by altering codon 385 of the wild type mouse FGFR4 gene or codon 386 of the wild-type rat FGFR4, such that codon 385 or 386, respectively, no longer encodes glycine. The construction of a gene with a 385th or 386^{th} codon, respectively, that does not encode glycine can be achieved by methods well known in the art.

Glycine is encoded by GGA, GGC, GGG, or GGT. A codon that does not encode glycine may be, for example, a codon that encodes Phe (TTT, TTC); Leu (TTA, TTG, CTT, CTC, CTA, CTG); Ile (ATT, ATC, ATA); Met (ATG); Asp (GAC, GAT); Ser (TCT, TCC, TCA, TCG), Val (GTT, GTC, GTA and GTG); Pro (CCT, CCC, CCA, CCG); Thr (ACT, ACC, ACA, ACG), Ala (GCT, GCC, GCA, GCG); His (CAT, CAC),Gln (CAA, CAG); Asn (AAT, AAC); Lys (AAA, AAG) ; Glu (GAA, GAG); Cys (TGT, TGC); Trp (TGG); Arg (CGT, CGC, CGA, CGG, AGA, AGG); Ser(AGT, AGC); Tyr (TAC, TAT) or one of the stop codons (TAA, TAG, TGA). Again, methods for the introduction of site-specific nucleic acid mutations are well known.

Alternatively, at least one codon of the wild-type FGFR4 may be altered such that it encode another amino acid than the wild-type amino acid as long as the modification, if present at least some or all or essentially all cells of the animals of the present invention, e. g., a rodent, in a heterozygous or homozygous manner, results in a phenotype associated with an alteration in tumor progression and/or formation as described herein, e. g., an increased rate of tumor growth and/or metastasis formation compared to a wild-type animal. In a preferred embodiment said animal additionally expresses in the genome of at least some of its cells a transgene encoding a TGF-α protein.

The nucleic acid sequences or genes encoding the modified FGFr4 proteins, and fragments thereof, of the invention may exist alone or in combination with other nucleic acid sequences, for example within episomal elements, genomes, or vector molecules, such as plasmids, including expression or cloning vectors.

The term "nucleic acid sequence" as used herein refers to any contiguous sequence series of nucleotide bases, i. e., a polynucleotide, and is preferably a ribonucleic acid (RNA) ordeoxy-ribonucleic acid (DNA). Preferably the nucleic acid sequence iscDNA. It may, however, also be, for example, a peptide nucleic acid (PNA).

An "isolated" nucleic acid molecule or gene, as referred to herein, is one, which is separated from other nucleic acid molecules ordinarily present in the natural source of the nucleic acid. Preferably, an "isolated" nucleic acid or gene is free of sequences, which naturally flank the nucleic acid (i. e., sequences located at the 5'-and 3'-termini of the nucleic acid) in the genomic DNA of the organism that is the natural (wild type) source of the DNA.

FGFR4 gene molecules can be isolated using standard hybridization and cloning techniques, as described, for instance, in Sambrook et al. (eds. ), MOLECULAR CLONING: A LABORATORY MANUAL (2nd Ed.), Cold Spring Harbor Laboratory Press, Cold Spring Harbor, NY, 1989 ; and Ausubel et al. (eds.), CURRENT PROTOCOLS IN MOLECULAR BIOLOGY, John Wiley & Sons, New York, NY, 1993.

A nucleic acid or gene of the invention can be amplified using cDNA, mRNA or, alternatively, genomic DNA, as a template and appropriate oligonucleotide primers according to standard polymerase chain reaction (PCR) amplification techniques. The nucleic acid or gene so amplified can be cloned into an appropriate vector and characterized by DNA sequence analysis. Furthermore, oligonucleotides corresponding to FGFR4 nucleotide sequences according to the invention can be prepared by standard synthetic techniques, e. g., using an automated DNA synthesizer.

Another aspect of the invention pertains to vectors, preferably expression vectors, containing a nucleic acid or gene encoding a modified FGFR4 protein, or derivatives, fragments, analogs, homologs or fusion proteins thereof. As used herein, the term "vector" refers to a nucleic acid molecule capable of transporting another nucleic acid to which it has been linked.

One type of suitable vector is a "plasmid", which refers to a circular double stranded circular DNA molecule into which additional DNA segments can be ligated. Another suitable type of vector is a viral vector, wherein additional DNA segments can be ligated into a viral genome or parts thereof. Certain vectors are capable of autonomous replication in a host cell into which they are introduced (e. g. , bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors".

A host cell of the invention, such as a prokaryotic or eukaryotic host cell in culture, can be used to produce (i. e., express) a modified FGFR4 protein as described herein.

Accordingly, the invention further provides a method for producing modified FGFR4 protein using the host cells of the invention. In one embodiment, the method comprises culturing the host cell of the invention (into which a recombinant expression vector encoding modified FGFR4 protein has been introduced) in a suitable medium such that modified FGFR4 protein is produced. In another embodiment, the method further comprises isolating modified FGFR4 protein, i. e., recombinantly produced protein, from the medium or the host cell.

The host cells of the invention can also be used to produce non-human transgenic animals. For example, in one embodiment, a host cell of the invention is a fertilized oocyte or an embryonic stem cell into which modified FGFR4 protein-coding sequences have been introduced. Such host cells can then be used to create non-human transgenic animals in which exogenous FGFR4 sequences have been introduced into their genome, or animals created by homologous recombination, in which endogenous FGFR4 sequences have been altered (see Examples, Material and Methods, below).

### Examples

Here we show in a genetically "clean" system the impact of a single nucleotide difference in the codon 385 of the mouse FGFR4 gene that converts a Glycine to an Arginine in the transmembrane domain of the receptor, on mammary cancer progression *in vivo.* In the following and in the Figures, the amino acid 385 of the mouse sequence (SEQ ID NO: 1) is named according to its corresponding amino acid position 388 in humans (cf. SEQ ID Nos:2 and 3). We generated a *FGFR4 Arg388 knock-in (KI)* mouse model in order to investigate the effect of the two different FGFR4 alleles on breast cancer progression. For this purpose we crossed the *FGFR4 Arg388 KI* mice to WAP-TGFα/EGFR transgenic mice (19,20). In this model, TGFα-overexpression is controlled by the whey acidic protein (WAP) promoter which specifically activates the transgene in mammary epithelial cells in mid-pregnancy (19). Thus, the process of mammary carcinogenesis is promoted by the over constitutively high expression of TGFα, a ligand of the epidermal growth factor receptor (EGFR). Overexpression of TGFα in mammary epithelial cells results in accelerated alveolar development and impaired cell differentiation leading to failures in female lactation. Moreover, mammary involution is delayed and some alveolar structures fail to regress completely. As a consequence these hyperplasic alveolar nodules increase in number with successive pregnancies, and in some cases progress to tumors of variable histotype (20).

Here we report that the substitution from Glycine to Arginin at codon 388 enhances the progression of breast cancer in the WAP-TGFα mouse mammary carcinoma model in mass and size of the occurring tumors and this progression in vivo could be confirmed by *in vitro* data generated in mouse embryo fibroblasts. Moreover the FGFR4 Arg388 allele promotes lung metastasis in size and number of the occurring metastases.

These results spotlight the importance of the FGFR4 Arg388 allele in human breast cancer progression and may therefore serve as a prognostic marker of clinical outcome for patients affected by this disease.

### Materials and methods

### Mouse Targeting Construct

The genomic sequence of the murine *FGFR4* was detected in the RPCI mouse PAC library 21 of SV/129 genetic background (Celera,USA) by a specific cDNA-probe detecting exon 8-10. Exon 2-12 of the murine *FGFR4* (12,5kb) was then cloned into a pBS-vector (Stratagene, California) via a Spel/Sacll restriction (Biolabs, New England). Afterwards the G to A single nucleotide polymorphism (SNP) was introduced via specific mutagenesis in a subfragment of 320bp, which was cloned into a pcDNA.3 vector (Invitrogen, USA) (21,22). This fragment containing the SNP was then recloned into the pBS-vector. The selection-cassette was finally integrated by Scal (Biolabs, New England) restriction. Prior to electroporation of embryonic stem cells the targeting construct was linearised by Sall (Biolabs, New England) restriction.

### Targeting of Embryonic Stem Cells (ES-cells) and Selection of Positive Clones

The ES-cell line E14 (23) was maintained on feeder-cells (i. e., irradiated mouse embryonic fibroblasts) in Dulbecco's modified Eagles Medium (DMEM, high glucose) containing 2mM Glutamine, 1000U/ml LIF, 0.1mM β-Mercaptoethanol and 20% heat-inactivated foetal calf serum.

For transfection, 4x10⁷ cells were mixed with 100µg of linearised targeting construct in PBS to a final volume of 800µl. Electroporation (240V, 500µF, 6 msec) was performed with a GenePulser (BioRad, Germany) and the cells were plated in 10 cm tissue culture dishes in DMEM containing 20% FCS,2mM Glutamin, 1000U/ml Lif and 0.1 mM β-Mercaptoethanol.

On the next day the cells were selected with 200µg/ml G418 for integration of the construct. Negative selection was done with 2µM gancyclovir.

Resistant clones were analyzed by Southern blotting (24) for homologous and illegitimate recombination via a 5' external-probe and a neomycin-specific probe, respectively. To generate chimeric mice, positive ES-cell clones were microinjected in C57BL/6 blastocysts and implanted into the uterus of a pseudo-pregnant recipient mother.

### Mice and Genotyping

Chimeric mice were backcrossed to C57BL/6 mice to raise a founder generation with germline transition. For removal of the neoR- selection cassette mice were crossed with Deleter-Cre transgenic mice. Cre-deleter mice were again backcrossed to C57BL/6 mice to generate the first generation of the *FGFR4 Arg388 knock-in (KI)* mice. *FGFR4 Arg388 KI* mice were backcrossed at least ten times to C57/BL6 mice or five times to FVB mice. *WAP-TGF*α/*EGFR* transgenic mice were obtained by L. Henninghausen, NIH, Bethesda, USA in a mixed C57BI/ 6 and FVB genetic background and were backcrossed to C57BL/6 mice ten times. Mice were kept in the animal facility of the Max-Planck-Institute of Biochemistry under normal conditions.

Genotype was determined by PCR of genomic tail-DNA isolated using the Qiagen Blood & Tissue DNeasy Kit according to the manufacturer's recommendation. The removal of the selection cassette was detected using neoR-specific primers (5'-AGGATCTCCTGTCATCTCACCTTCCTCCTG-3' and 5'-AAGAACTCGTCAAGAAGGCGATAGAAGGCG-3'). Removal of the Cre transgene was determined by Cre-specific primers (5'-AACATGCTTCATCGTCGG-3' and 5'-TTAGGATCATCAGCTACACC-3'). Primer for detecting the genotype of the *FGFR4* allele were specific for amplifying a 168bp band spanning the *FGFR4-SNP* (forward: 5'-CGTGGACAACAGCAACCCCTG-3'; reverse: 5'-GCTGGCGAGAGTAGTGGCCACG-3') with subsequent restriction of the amplification product via Mval restriction enzyme to distinguish the different *FGFR4* alleles. The presence of the TGFα-transgene was confirmed by performing PCR analysis with TGF-α forward 5'-TGTCAGGCTCTGGAGAACAGC-3' and reverse 5'-CACAGCGAACACCCACGTACC-3' primers (primer sequence provided by L.
Henninghausen, NIH, Berthesda, USA).

To analyse the occurring tumors, mice were sacrificed by cervical dislocation and opened ventrally. All mammary glands were excised for tumor-measurement. Tumor size and mass were analysed by metrical measurement and weighing of the tumor tissue and the mammary gland tissue independently. Raw-data were normalised to bodyweight. All data are shown as mean ± SDM. All p-values were calculated using the paired students T-Test and values < 0.05 were considered statistically significant.

### Mouse Embryonic Fibroblasts (MEFs)

MEFs were isolated from 13.5 dpc embryos and kept in DMEM containing 10% FCS and maintained following the 3T3 protocol (25).

To perform a proliferation assay, 1x 10⁵ MEFs were seeded in 6cm-dishes, maintained to 80% confluence, counted and re-seeded till senescence. The population doubling rate was determined by log (N/N0 x 3,33 (N = cells at the end of growth period; **N0** = number of cells plated).

Senescence assays (Cell Signalling, USA) were performed on 1x 10⁵ cells seeded in 6cm-dishes. After 24h cells were stained for β-galactosidase expression according to the manufacturer's recommendation and analysed under a light microscope (Visitron Systems, Zeiss).

The Focus Formation Assay was performed by infection (26) of MEFs with pLXSN (Clontech, Palo Alto,USA) based retroviruses containing the oncogenes v-src (positive control) HER2, EGFR or c-Kit. 24h after infection cells were starved in medium containing 4% FCS and maintained for 21 days. Afterwards cells were stained with crystal violet and foci were counted macroscopically.

Transmigration of MEFs were analysed in Boyden Chambers (Schubert & Weiss, Germany). 1.5 x 10⁴ cells were seeded in starving medium containing 0% FCS.

Migration was performed to DMEM containing 4% FCS for 16h. Afterwards cells were stained with crystal violet and migrated cells were analysed macroscopically. To perform an apoptosis assay 1.5 x 10⁴ cells in DMEM containing 10% FCS were seeded in 12-well plates. After 24h cells were treated with 0.5µM doxorubicin for 48 h. Afterwards cells were stained with propidium-iodide and apoptotic cells were determined in a FACS (FACScalibus, BD) analysis as previously described (27) .

### RNA and Light Cycler® Analysis

Total RNA of minced murine tissues of adult mice was isolated using the RNeasy Kit (Qiagen, Germany) according to the manufacturer's recommendation. The quality of the isolated RNA was confirmed by agarose-gel electrophoresis visualising the 16S and 18S RNA. RNA was reverse transcribed into cDNA via the first strand cDNA kit of Boehringer Mannheim according to the manufacturer's protocol. The obtained cDNA was analysed via Light Cycler® Technology (Roche Diagnostics, Mannheim) for FGFR4 expression levels. Raw-data were normalised on expression levels of the housekeeping-gene HPRT and plotted as absolute values. Data are shown as mean ± SDM.

### Immunoprecipitation and Western Blotting

For preparation of protein lysates, tumor samples were snap-frozen in liquid nitrogen, minced by an Ultratorax (Janke & Kunkel, IKA Labortechnik), lysed in RIPA lysis buffer containing phosphatase and proteinase-inhibitors for 30 min and precleared by centrifugation. Cultured cells were lysed in RIPA Buffer containing phosphatase and proteinase-inhibitors. For immunoprecipitation, lysates (1000mg protein) were incubated with Protein A sepharose beads (GE Healthcare, San Francisco) and the according primary antibody (α-FGFR4 H121, Santa Cruz) at 4°C over night. Afterwards samples were subjected for Western Blotting as previously described (28).

The following primary antibodies were used: FGFR4 sc9006 (Santa Cruz), 4G10 (upstate), α-actin/ α-tubulin (Sigma); secondary antibodies: α-rabbit-HRP conjugated (BioRAD ) and α-mouse-HRP conjugated (Sigma)

### Histology and Immunohistochemistry

Tumor samples and tissues were fixed in 70 % Ethanol at 4°C overnight. On the next day samples were embedded in paraffin and sections of 4-8µM were cut on a microtome (HM355S, microm). The sections were subjected to deparaffinisation in xylene and rehydrated in a graded series of ethanol. Antigen retrieval was achieved by cooking in citrate buffer (pH 6) in a microwave. Immunohistochemical staining was done with the Vectastain Staining Kit (Vector Laboratories, Burlingame) following the manufacturer's protocol. After blocking with 10% horse serum in PBS buffer containing 3% Triton-X for one hour, the sections were incubated with the primary antibody (α-FGFR4 Hs121, Santa Cruz) at 4°C overnight. The secondary antibody (α-rabbit, VectorLabs, USA) was incubated for one hour in PBS buffer containing 3% Triton-X. Mayer's Hematoxylin (Fluka, Switzerland) was used as counterstain.

For pathological analysis and quantitation of metastases, lungs were sectioned and analysed at 800 to 1000 µm intervals. Sections were stained with hematoxilin and eosin (H&E, Fluka, Switzerland) to identify lung metastases under the light microscope. Metastatic burden was calculated based on number and size of metastatic nodules.

### Results:

### Generation of FGFR4 Arg388 KI and FGFR4 Arg388 WAP-TGFα Transgenic Mice

Since an impact of the human *FGFR4 Arg388* allele on tumor progression was just shown in correlative and partially controversial clinical studies there was an urgent need to ultimately clarify the influence of this single nucleotide polymorphism (SNP) on tumor progression *in vivo.* Here, the defined genetic background of a generated mouse model overcomes the heterogeneity of patient cohorts and thus the cause of resulting conflictive conclusions. Therefore, we generated a *FGFR4 Arg388* knock- in (KI) model in the genetic background of SV/ 129 mice, which represents the first directly targeted KI mouse model to investigate the impact of a single nucleotide polymorphism on the progression of cancer. To achieve the genomic sequence of the murine FGFR4, a BAC-library was screened using a specific cDNA-probe detecting exons 8-10 of the FGFR4 gene and positive clones were analyzed by Southern blotting (data not shown). The gene targeting construct (Figure 1 A) contains exons 2-12 (12.5 kb) of the genomic sequence of the murine *FGFR4.* In order to generate the *FGFR4 Arg388* allele, the glycine in exon 8 was changed to an arginine by site-directed mutagenesis. A neomycin selection cassette flanked by loxP sites was cloned between exons 10 and 11. After gene targeting upcoming neomycin-resistant ES-cell clones were analysed by Southern blotting (Figure 1B 1). Here a 5'-external probe was used to detect correct homologous recombination identifiable by an additional 10kb band. The genotype of the positive ES-cell clones was then analysed by PCR-RFLP of the genomic DNA. Since the SNP inserts a new restriction site, the obtained amplification product in *FGFR4 Arg388* carrying clones could be cut via the *Mval* restriction enzyme resulting in an additional 93bp fragment (Figure 1B 2).

Next, positive clones were injected into blastocysts of pseudo-pregnant mice to generate chimeras. These mice were then backcrossed to C57BL/6 mice to raise the first generation of *FGFR4 Arg388* KI mice. In order to delete the neomycin selection cassette, the FGFR4 Arg 388 mice were crossed to mice transgenic for the Cre-recombinase (Deleter-Cre).

*FGFR4 Arg388 KI* Cre-deleted mice were analysed by segregation analysis of a statistically significant number of mice for mendelian inheritance of the *FGFR4* allele (Figure 1 C). In backcrosses to *FGFR4 GlylGly388* mice, the offspring displayed the expected distribution of 1:1 from *FGFR4 GlylGly388* to *FGFR4 Glyl Arg388.* Heterozygote intercrosses displayed the expected distribution of 1:2:1 from *FGFR4 GlylGly388* to *GlylArg388* to *Arg*/*Arg388.* Hence, the FGFR4 Arg388 allele is inherited in the correct mendelian ratio.

To investigate the impact of the FGFR4 Arg388 on mammary cancer progression, the *FGFR4 Arg388 KI* mice were crossed to mice transgenic for WAP-TGFα/EGFR (Figure 1D 3). To ensure normal lactation of female mice the transgene was only inherited by males. To confirm the presence of the TGFα transgene in the progeny we performed genotyping with specific primers for exogenous TGFα (Figure 1D 2). To distinguish the different FGFR4 alleles, the genotyping was done by PCR-RFLP by the aforementioned additional restriction site (Figure 1D 1).

### FGFR4 Arg388 KI Mice mimic their human Counterparts

In humans, the *FGFR4 Arg388* allele is expressed in various tissues without any difference compared to the *FGFR4 Gly388* and has yet no known impact on the organism itself (11). Similarly, the *FGFR4 Arg388 KI* mouse model displays no yet known obvious phenotype that distinguishes the *Arg388* from *Gly388* carrying mice (data not shown). To check if the generated *FGFR4 Arg388 KI* mice mimic their human counterpart also in FGFR4 expression, localisation and distribution, we analyzed FGFR4 mRNA- and protein- levels and analysed the localisation and distribution in various tissues of adult mice.

As shown in Figure 2A and B, FGFR4 is expressed in various tissues including mammary gland, lung, brain or kidney. No difference between the different FGFR4 alleles on mRNA as well as on protein expression level is detectable. Next, we analysed the expression and localisation of FGFR4 in different tissues immunohistochemically (Figure 2 C). Here FGFR4 is detectable in various tissues and as with mRNA or protein expression-levels, there is no difference between the *FGFR4* alleles. Two examples for FGFR4-stained tissues are given in Figure 2D. In the lung, FGFR4 is expressed in smooth muscles, blood vessels and bronchial epithelial cells. In the mammary gland, blood vessels and ductal epithelial cells show a clear FGFR4 staining. These results are matching previously published data on human samples (29).

Hence the *FGFR4 Arg388 KI* mice seem to mimic their human counterparts in mRNA and protein expression levels, localisation and distribution.

### FGFR4 Arg388 promotes Tumor Progression

Previous reports of clinical studies do not implicate the *FGFR4 Arg388* allele in tumor initiation, but rather associate it with enhanced disease progression once cancer has been initiated (11,12). Therefore we crossed the FGFR4 Arg388 KI mice to mice transgenic for WAP-TGFα/EGFR to initiate mammary tumors and to investigate for the first time the impact of the SNP *FGFR4 Arg388* allele on mouse mammary tumor progression *in vivo.*

For this purpose we analysed the mass, area and percentage of the occurring tumors (Figure 3 A-D). Here, the tumor mass and area is significantly (*GlylArg*-p= 0.03; *ArglArg*-p= 0.002) increased (*GlylArg*-p= 0.01; *ArglArg*-p= 0.0006) in *FGFR4 Arg388 (Arg388)* carrying mice transgenic for WAP-TGFα when compared to *FGFR4 Gly388 (Gly388)* controls (Figure 3 A and B). These results indicate that the *FGFR4 Arg388* allele is a potent enhancer of TGFα-induced mammary tumors in mass and area.

Further we investigated the impact of the FGFR4 Arg388 allele on the initiation of mammary tumors in the WAP-TGFα/EGFR model. To do that we analyzed the amount of transformed mammary gland epithelia which is the percentage of mass and area of the tumor compared to the whole mammary gland (Figure 3C and 3 D). Here the percentage of the tumor mass is significantly increased (*GlylArg*-p= 0.05; *ArglArg*-p= 0.005) whereas the percentage of tumor area shows a significant increase only when *GlylGly and ArglArg* (p= 0.002) mice are compared. Thus, the *FGFR4 Arg388* allele not only promotes tumor growth as shown in Figure 3A and B but seems to facilitate the initiation of TGFα/EGFR -induced mammary tumors since the percentage of tumorigenic mass and area increases from heterozygous to homozygous *Arg388* carrying mice. Furthermore, the potent tumor enhancing impact of the FGFR4 Arg388 allele is in evidence by comparing an Arg/Arg 388 carrying mouse to a Gly/Gly control sacrificed after 6 month of mating and thereby tumor progression (Figure 3 E). In contrast, the inability of the FGFR4 to influence cancer initiation by itself is shown in Figure 7 A/B.

To further investigate the underlying mechanism of the tumor enhancing effect of the FGFR4 Arg388 allele, we studied possible molecular differences of the FGFR4 alleles. In many human cancers overexpression of the FGFR4 is a commonly observed feature of tumors (1, 4, 30, 31). To examine the FGFR4 expression in TGFα/EGFR -derived tumors we first analysed the expression by immunoprecipitation of the FGFR4 from tumor lysates of every genotype. Here, the FGFR4 protein is clearly overexpressed in tumors, when compared to noncancerous mammary gland, however, there is no detectable difference between the different alleles (Figure 3 F).

Furthermore, we analysed the constitutive phosphorylation status of the FGFR4 to check if the Arg388 allele has any influence on the kinase activity and thereby leading to a tumor promoting effect. As shown in Figure 3F, FGFR4 Arg/Arg388 seems to be more phosphorylated and thereby more activated then Gly/Gly- or Gly/Arg388, a possible hint of the tumor promoting potential of the FGFR4 Arg388 allele. The activation of downstream molecules like Erk or Akt did not show significant differences between the expressed FGFR4 alleles (data not shown). We also checked the expression levels of FGFR4 of every genotype in murine samples immunohistochemically (Figure 3 G). Remarkably, the expression of the FGFR4 alleles displays no differences in adenocarcinomas (Figure 3 G 2) but show a clear increased expression of the Arg388 allele compared to Gly 388 in hyperplasic mammary glands (Figure 3G 1). Maybe the overexpression of FGFR4 Arg388 in oncogenesis is accelerated relative to the Glycine allele and thereby promotes tumor progression with an earlier onset. Additionally, the given examples of TGFα/EGFR-derived tumors (Figure 3 G) indicate no pathohistological differences due to FGFR4 Arg388 allele expression.

### FGFR4 Arg388 decreases Time Point of Tumor Incidence and promotes Tumor Progression over time

To further analyse the tumor promoting effect of FGFR4 Arg388 we followed the tumor progression of all three genotypes over time in the WAP-TGFα/EGFR model.

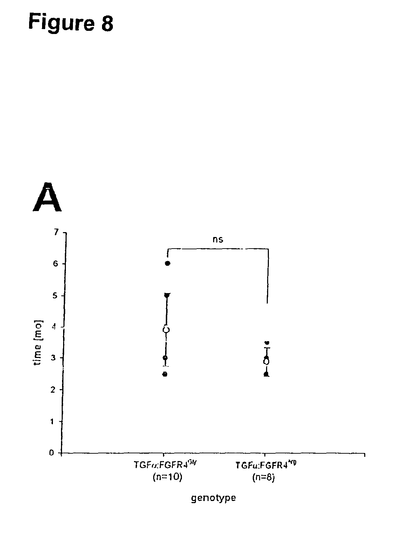

First we checked the visible time point of tumor incidence. As shown in Figure 4A the *FGFR4 Arg388* (*GlylArg388 and ArglArg388* were pooled) carrying mice significantly (p= 0.001) develop tumors at earlier time points than the FGFR4 Gly388 controls. In addition, we further checked the time point of tumor onset in *FGFR Arg388 KI* mice compared to Gly-carriers induced by the WAP-TGFα transgen in the FVB background. Similarly, Arg allele-carrying mice display an earlier onset of tumors (p= ns) as shown in Figure 8 A.

Further, *FGFR4 GlylArg388-* or *ArglArg388* mice establish not only a larger amount of tumors simultaneously, but importantly, increase their number of tumors over time faster than *FGFR4 Gly*/*Gly388* mice (Figure 4 B). The tumor mass and area of *FGFR4 GlylArg388* and *Arg*/*Arg388*-mice also progresses considerably faster than that of *FGFR4 GlylGly388*-mice (Figure 4C and D). Remarkably, the *FGFR4 Arg388* carrying mice show no difference at early time points of tumor progression, but mice homozygous for *Arg388* show an immense acceleration of tumor growth after 6-8 months. Hence, a heterozygous *FGFR4 Arg388* status seems to be sufficient for a decreased tumor incidence, but homozygous Arg388 carriers display an obvious faster progression once tumor formation occurs.

Next, we analysed the share of tumor mass and area in the mammary gland over time (Figure 4E and F). Here, both the percentage of tumor mass and size shows a clear increase from *FGFR4 GlylGly388* to *GlylArg388* to *ArglArg388* mice. These data confirm the fact that the Arg388 allele seems to facilitate tumor initiation of WAP-TGFα/EGFR -induced breast tumors.

In summary, the *FGFR4 Arg388* allele promotes breast tumor progression over time in number, mass and size of the occurring tumors and seems to facilitate the initiation of oncogenesis and thereby decrease the time point of tumor onset.

### FGFR4 Arg388 promotes Cancer Cell Metastasis

As clinical outcome of cancer is dependent on the invasive stage of the primary tumor it is essential to investigate the impact of the *FGFR4 Arg388* allele on aggressiveness and invasiveness of WAP-TGFα/EGFR-derived tumors. Therefore we investigated the lungs of the dissected mice for the occurrence of distant metastases. Strikingly, *FGFR4 Arg388* mice show an earlier incidence of lung metastases when compared to *Gly388* mice (Figure 5 A) but do not differ pathohistologically (Figure 5 B). Beyond this, the *FGFR4 Arg388* allele enhances lung metastases in both, size and number when compared to *FGFR4 Gly*/*Gly388* to heterozygous to homozygous *FGFR4* Arg388-carrying mice (Figure 5 C). The highest differences are visible in the number of micro metastases lower than 80µm. These results suggest that the FGFR4 Arg388 allele seems to facilitate tumor cell invasion. Second, in metastases bigger than 360 µm, an evidence for an earlier incidence and a possible faster growth of metastases in *FGFR4 Arg388* carrying mice is observed.

### FGFR4 Arg388 promotes Cellular Transformation

To further investigate the possible mechanism of the tumor progressive impact of the *FGFR4 Arg388 allele* on tumor progression *in vitro* we performed focus formation assays on isolated E13.5 mouse embryonic fibroblasts (MEFs).

The cells were transformed with several oncogenes to check whether the results obtained from the *FGFR4 Arg388 KI* mice are conform *in vitro* and whether there is a hint for the mechanism of the tumor progressive effect. MEFs expressing *FGFR4 GlylGly388, GlylArg388* or *Arg*/*Arg388* were infected with HER2, EGFR or c-kit while v-src served as a positive control. As shown in Figure 6A the number of foci in *FGFR4 Arg388* MEFs is markedly increased in all three oncogenes suggesting that the *FGFR4 Arg388* allele promotes cell transformation in cooperation with classical oncogenes. Remarkably, cell transformation by the EGFR or c-kit, receptors, which are normally regarded as weak oncogenes, lead to an unusual high number of foci. A possible explanation for this phenomenon may be that there is yet unknown crosstalk between *FGFR4 Arg388* and other receptor tyrosine kinases, which should be the subject of future investigations.

To mimic oncogenesis over time we performed an additional Focus Formation assay by terminating the focus formation at different time points (Figure 6 B). It is clearly shown that Arg/Arg- MEFs not only transform considerably faster, but also generate an increased number of foci. Hence the enhanced and more intense progression over time *in vivo* could be confirmed *in vitro.*

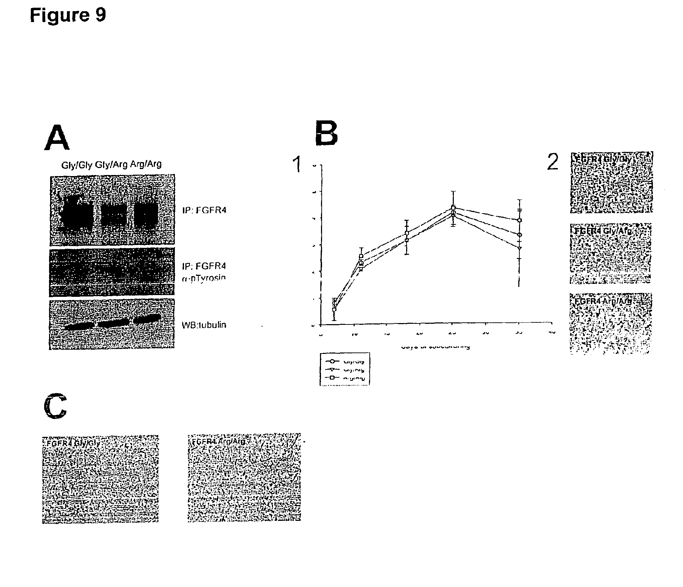

To support these observations by molecular analytical methods we determined whether the FGFR4 Arg388 allele is hyperactivated in MEFs and thereby enhances cell transformation. The expression of the different FGFR4 isoforms is equal in MEFs as well as its basic state of phosphorylation (Figure 9 A). Next, we wanted to check if *FGFR4 Arg388* facilitates cell survival or influences physiological processes of MEFs. Therefore we analysed the number of population doublings until the cells enter senescence *in vitro* and additionally stained MEFs, subcultured for 30 days, for β-galactosidase to visualize senescent cells. However, we found no obvious differences between the different FGFR4 alleles (Figure 9 B). Hence, the FGFR4 Arg388 allele does not seem to promote a prolonged cellular lifespan. Further we wanted to investigate the influence of the FGFR4 Arg388 allele on physiological processes. As a motility enhancing effect of the FGFR4 Arg388 allele had already been shown by Bange and colleagues with a human mammary carcinoma cell line, we wanted to confirm this observation in MEFs, but as shown in Figure 9C no difference observed when Gly/Gly- were compared to Arg/Arg-MEFs. Contrarily, Arg-carrying MEFs seemed to strongly support cell survival in response to DNA-damaging agents. After 48h of treatment with doxorubicin, Arg/Arg- MEFs displayed a significantly (p= 0.03) decreased percentage of apoptotic cells compared to Gly/Gly -MEFs. Additionally, we investigated the anti-apoptotic impact of the FGFR4 Arg388 after treatment with other chemotherapeutic drugs. In the presence of 3 µM cisplatin, that, similar to doxorubicin, intercalates with DNA, Arg-carrying MEFs show decreased apoptosis after 48hrs of treatment. Contrarily, after 48hrs of treatment with taxol, which interferes with the organisation of the mitotic spindle, no differences between Gly-or Arg-carrying MEFs were observed (Figure 6 C).

Maybe, the FGFR4 Arg388 allele enables the cells to survive DNA-damage by higher tolerance to chemotherapeutic drugs and DNA-damage or -repair systems respond faster and more effectively in MEFs expressing the FGFR4 Arg388 allele.

### Discussion

In this study we investigated for the first time the impact of the receptor tyrosine kinase FGFR4 alleles at codon 388 on the initiation and progression of breast cancer *in vivo.* The FGFR4 Arg388 KI per se mimics its human counterpart in expression, localisation and distribution of the FGFR4 and displays no yet known obvious phenotype. To investigate the role of the FGFR4 Arg388 knock-in mouse in the progression of mammary carcinoma we crossed the FGFR4 Arg388 mice to mice transgenic for WAP-TGFα/EGFR. We show that the FGFR4 Arg388 allele directly promotes occurring TGFα-induced mammary tumors in mass and size. In addition, these tumors also increase over time depending on the different FGFR4 genotypes. Furthermore, it decreases the visible time point of tumor incidence and therefore seems to facilitate tumor initiation which is demonstrated by a higher percentage of tumorigenic mass and size in the progression over time.

Remarkably, the FGFR4 Arg388 allele not only promotes aggressiveness but also supports invasiveness of lung metastases. The time point of metastases is substantially decreased and the lungs in FGFR4 Arg388 carrying mice are more invaded than in control animals.

These data strongly associate the FGFR4 Arg388 allele with poor prognosis and thereby highlight the receptor as a possible marker of breast cancer progression. Our *in vivo* results are in line with several clinical reports that were published since the discovery of the FGFR4 Arg388 allele by Bange and colleagues, which associate the FGFR4 Arg388 allele with tumor progression in various cancers like head and neck, prostate or breast (12-14).

In addition, our data in mice could be confirmed *in vitro.* Mouse embryonic fibroblasts carrying the Arg388 allele showed a higher transformation rate than control fibroblasts when infected with different oncogenes in a Focus Formation Assay. Furthermore, we could show a clear increase in number and growth rate of transformed foci in Arg388 MEFs over time.

But for all that, the molecular mechanism of the tumor progressive impact is still unknown. Our study provides some evidence that may be an explanation for the tumor progressive function of the FGFR4 Arg388 allele. First, the FGFR4 Arg388 allele was more activated in analysed tumors when compared to the other alleles probably leading to a more intensive signalling and further to a higher cell proliferation and tumor progression. Second, the unusual number of foci in c-kit-and EGFR-induced Focus Formation Assays. Maybe the FGFR4 Arg388 allele enables additional unknown crosstalk to other receptors and their downstream signalling molecules which can drive tumor progression. Third, the Gly388 allele was described to have a suppressive function (33). In this context it may be that the Arg388 allele fails to achieve this suppression and thereby releases or potentiates signalling of other receptor tyrosine kinases. Mass spectrometric analysis could be used to define either additional binding partners and adaptor proteins or additional downstream-molecules, which get activated after specific stimulation of the different FGFR4 alleles.

Exceptionally, we could show that Arg-carrying MEFs display an increased survival in response to DNA-damaging agents like doxorubicin. Maybe the FGFR4 Arg388 enables the cell to survive DNA-damaging and occurring genomic instability, which is a typical event in cellular transformation. If FGFR4 Arg388 expressing cells could easily deal genomic instability, they could consequently easier transform. Further, the FGFR4 Arg388 eventually supports DNA-repair mechanisms and thereby permits a faster and more effective DNA-repair. Hence a lower percentage of cells would enter apoptosis in response to DNA-damage.

However, our data suggest the FGFR4 Arg388 allele is a potent enhancer of breast tumor development and progression *in vivo.* Hence, further studies on our generated knock-in mouse should investigate a possible impact of the FGFR4 Arg388 allele also in other cancers, for example liver cancer. Here, several recent publications implicate the FGFR4 in liver functions and homeostasis (34). Further the development of a therapeutic antibody blocking the FGFR4 could possibly be used additionally with classical cancer therapies like chemotherapeutic drugs. Furthermore, the FGFR4 could not only be additionally targeted, but the genomic disposition of this receptor would also be conceivable to be included in the decision of cancer therapy with the according patient. This notion is also strongly supported by Thussbass and colleagues, who could show that a different time of relapse of treated mammary tumors after different drug-treatments is associated with the different FGFR4 alleles (16).

Recapitulatory, the implication of the FGFR4 and especially the tumor progressive impact of the Arg388 allele can not be negated. Our report suggests a role of the FGFR4 Arg388 allele as a marker for poor clinical outcome in breast cancer progression and metastasis. Furthermore, these observations highlight the impact of germline alteration and especially single nucleotide substitutions in receptor tyrosine kinase genes for the clinical progression of cancer and thereby validate FGFR4 as possible target for the development of prototypical drugs for individualized cancer therapies.

### References

1. Jeffers, M., LaRochelle, W.J., and Lichenstein, H.S. 2002. Fibroblast growth factors in cancer: therapeutic possibilities. Expert Opin Ther Targets 6:469-482.
2. Burke, D., Wilkes, D., Blundell, T.L., and Malcolm, S. 1998. Fibroblast growth factor receptors: lessons from the genes. Trends Biochem Sci 23:59-62.
3. Eswarakumar, V.P., Lax, I., and Schlessinger, J. 2005. Cellular signaling by fibroblast growth factor receptors. Cytokine Growth Factor Rev 16:139-149.
4. Gowardhan, B., Douglas, D.A., Mathers, M.E., McKie, A.B., McCracken, S.R., Robson, C.N., and Leung, H.Y. 2005. Evaluation of the fibroblast growth factor system as a potential target for therapy in human prostate cancer. Br J Cancer 92:320-327.
5. Morrison, R.S., Yamaguchi, F., Bruner, J.M., Tang, M., McKeehan, W., and Berger, M.S. 1994. Fibroblast growth factor receptor gene expression and immunoreactivity are elevated in human glioblastoma multiforme. Cancer Res 54:2794-2799.
6. Cappellen, D., De Oliveira, C., Ricol, D., de Medina, S., Bourdin, J., Sastre-Garau, X., Chopin, D., Thiery, J.P., and Radvanyi, F. 1999. Frequent activating mutations of FGFR3 in human bladder and cervix carcinomas. Nat Genet 23:18-20.
7. Jang, J.H., Shin, K.H., and Park, J.G. 2001. Mutations in fibroblast growth factor receptor 2 and fibroblast growth factor receptor 3 genes associated with human gastric and colorectal cancers. Cancer Res 61:3541-3543.
8. Ameyaw, M.M., Tayeb, M., Thornton, N., Folayan, G., Tariq, M., Mobarek, A., Evans, D.A., Ofori-Adjei, D., and McLead, H.L. 2002. Ethnic variation in the HER-2 codon 655 genetic polymorphism previously associated with breast cancer. J Hum Genet 47:172-175.
9. Przybylowska, K., Smolarczyk, K., Blasiak, J., Kulig, A., Romanowicz-Makowska, H., Dziki, A., Ulanska, J., and Pander, B. 2001. A C/T polymorphism in the urokinase-type plasminogen activator gene in colorectal cancer. J Exp Clin Cancer Res 20:569-572.
10. Morimoto, Y., Ozaki, T., Ouchida, M., Umehara, N., Ohata, N., Yoshida, A., Shimizu, K., and Inoue, H. 2003. Single nucleotide polymorphism in fibroblast growth factor receptor 4 at codon 388 is associated with prognosis in high-grade soft tissue sarcoma. Cancer 98:2245-2250.
11. Bange, J., Prechtl, D., Cheburkin, Y., Specht, K., Harbeck, N., Schmitt, M., Knyazeva, T., Muller, S., Gartner, S., Sures, I., et al. 2002. Cancer progression and tumor cell motility are associated with the FGFR4 Arg(388) allele. Cancer Res 62:840-847.
12. Streit, S., Bange, J., Fichtner, A., Ihrler, S., Issing, W., and Ullrich, A. 2004. Involvement of the FGFR4 Arg388 allele in head and neck squamous cell carcinoma. Int J Cancer 111:213-217.
13. Streit, S., Mestel, D.S., Schmidt, M., Ullrich, A., and Berking, C. 2006. FGFR4 Arg388 allele correlates with tumor thickness and FGFR4 protein expression with survival of melanoma patients. Br J Cancer 94:1879-1886.
14. Wang, J., Stockton, D.W., and Ittmann, M. 2004. The fibroblast growth factor receptor-4 Arg388 allele is associated with prostate cancer initiation and progression. Clin Cancer Res 10:6169-6178.
15. Spinola, M., Leoni, V., Pignatiello, C., Conti, B., Ravagnani, F., Pastorino, U., and Dragani, T.A. 2005. Functional FGFR4 Gly388Arg polymorphism predicts prognosis in lung adenocarcinoma patients. J Clin Oncol 23:7307-7311.
16. Thussbas, C., Nahrig, J., Streit, S., Bange, J., Kriner, M., Kates, R., Ulm, K., Kiechle, M., Hoefler, H., Ullrich, A., et al. 2006. FGFR4 Arg388 allele is associated with resistance to adjuvant therapy in primary breast cancer. J Clin Oncol 24:3747-3755.
17. Jezequel, P., Campion, L., Joalland, M.P., Millour, M., Dravet, F., Classe, J.M., Delecroix, V., Deporte, R., Fumoleau, P., and Ricolleau, G. 2004. G388R mutation of the FGFR4 gene is not relevant to breast cancer prognosis. Br J Cancer 90:189-193.
18. Spinola, M., Leoni, V.P., Tanuma, J., Pettinicchio, A., Frattini, M., Signoroni, S., Agresti, R., Giovanazzi, R., Pilotti, S., Bertario, L., et al. 2005. FGFR4 Gly388Arg polymorphism and prognosis of breast and colorectal cancer. Oncol Rep 14:415-419.
19. Pittius, C.W., Hennighausen, L., Lee, E., Westphal, H., Nicols, E., Vitale, J., and Gordon, K. 1988. A milk protein gene promoter directs the expression of human tissue plasminogen activator cDNA to the mammary gland in transgenic mice. Proc Natl Acad Sci U S A 85:5874-5878.
20. Sandgren, E.P., Schroeder, J.A., Qui, T.H., Palmiter, R.D., Brinster, R.L., and Lee, D.C. 1995. Inhibition of mammary gland involution is associated with transforming growth factor alpha but not c-myc-induced tumorigenesis in transgenic mice. Cancer Res 55:3915-3927.
21. Messing, J. 1983. In vitro DNA synthesis as a tool to analyze and alter genes. Basic Life Sci 25:9-15.
22. Kunkel, T.A. 1985. The mutational specificity of DNA polymerases-alpha and - gamma during in vitro DNA synthesis. J Biol Chem 260:12866-12874.
23. Hooper, M., Hardy, K., Handyside, A., Hunter, S., and Monk, M. 1987. HPRT-deficient (Lesch-Nyhan) mouse embryos derived from germline colonization by cultured cells. Nature 326:292-295.
24. Southern, E.M. 1974. An improved method for transferring nucleotides from electrophoresis strips to thin layers of ion-exchange cellulose. Anal Biochem 62:317-318.
25. Todaro, G.J., and Green, H. 1963. Quantitative studies of the growth of mouse embryo cells in culture and their development into established lines. J Cell Biol 17:299-313.
26. Pear, W.S., Nolan, G.P., Scott, M.L., and Baltimore, D. 1993. Production of high-titer helper-free retroviruses by transient transfection. Proc Natl Acad Sci U S A 90:8392-8396.
27. Nicoletti, I., Migliorati, G., Pagliacci, M.C., Grignani, F., and Riccardi, C. 1991. A rapid and simple method for measuring thymocyte apoptosis by propidium iodide staining and flow cytometry. J Immunol Methods 139:271-279.
28. Wakabayashi, Y., Mao, J.H., Brown, K., Girardi, M., and Balmain, A. 2007. Promotion of Hras-induced squamous carcinomas by a polymorphic variant of the Patched gene in FVB mice. Nature 445:761-765.
29. Partanen, J., Makela, T.P., Eerola, E., Korhonen, J., Hirvonen, H., Claesson-Welsh, L., and Alitalo, K. 1991. FGFR-4, a novel acidic fibroblast growth factor receptor with a distinct expression pattern. Embo J 10:1347-1354.
30. Ezzat, S., Zheng, L., Zhu, X.F., Wu, G.E., and Asa, S.L. 2002. Targeted expression of a human pituitary tumor-derived isoform of FGF receptor-4 recapitulates pituitary tumorigenesis. J Clin Invest 109:69-78.
31. Jaakkola, S., Salmikangas, P., Nylund, S., Partanen, J., Armstrong, E., Pyrhonen, S., Lehtovirta, P., and Nevanlinna, H. 1993. Amplification of fgfr4 gene in human breast and gynecological cancers. Int J Cancer 54:378-382.
32. Guy, C.T., Cardiff, R.D., and Muller, W.J. 1992. Induction of mammary tumors by expression of polyomavirus middle T oncogene: a transgenic mouse model for metastatic disease. Mol Cell Biol 12:954-961.
33. Stadler, C.R., Knyazev, P., Bange, J., and Ullrich, A. 2006. FGFR4 GLY388 isotype suppresses motility of MDA-MB-231 breast cancer cells by EDG-2 gene repression. Cell Signal 18:783-794.
34. Huang X, Yang C, Luo Y, Jin C, Wang F, McKeehan WL. FGFR4 prevents hyperlipidemia and insulin resistance but underlies high-fat diet induced fatty liver. Diabetes, Oct 2007.

## Claims

1. A rodent animal comprising an endogenous gene encoding a modified FGFR4 protein, wherein the modification is an amino acid substitution in the wild-type FGFR4 of said rodent at the amino acid position corresponding to amino acid position 385 of SEQ ID NO: 1.

2. The animal of claim 1 wherein the rodent is a mouse, hamster or rat, preferably a mouse.

3. The animal according to any one of claims 1-2, wherein said amino acid substitution is with an amino acid with a charged side chain, preferably a lysine, arginine or histidine, and more preferably an arginine.

4. The animal according to any one of claims 1-3, wherein the modified FGFR4 has the amino acid sequence of SEQ ID NO: 5 or SEQ ID NO: 6.

5. The animal according to any one of claims 1-4, wherein at least some of its cells or all cells of said non-human animal comprise said endogenous modified FGFR4 encoding gene.

6. The animal according to any one of claims 1-5, wherein the at least some cells or all cells are heterozygous or homozygous with respect to said modified FGFR4.

7. The animal according to any one of claims 1-6, additionally displaying uncontrolled cell growth, preferably cancer and/or metastasis formation.

8. The animal according to any one of claims 1-7, further being irradiated, treated with cancer-inducing agent and/or comprising a transgene, wherein said transgene comprises an oncogene.

9. The animal according to claim 8 wherein
(a) the oncogene is TGF-α, TGF-β, erb-B2, p53, myc, or ras; and/or
(b) the cancer-inducing agent is dimethylhydrazine (DMH), azoxymethane (AOM), N-methyl-N-nitro-N-nitrosoguanidine (MNNG), N-methyl-N-nitrosourea (MNU), ethyl-nitroso-urea (ENU) or 12-0-tetradecanoylphorbol-13-acetate (TPA).

10. The animal according to any one of claims 8 or 9, wherein said transgene is expressed in mammary cells.

11. The animal of any one of claims 1-10 wherein the modification of said FGFR4 results in a phenotype associated with an alteration in tumor progression and/ or formation.

12. The animal of claim 10 wherein the alteration in tumor progression is **characterized by** an increased rate of tumor growth and/or metastasis formation compared to a wild-type animal.

13. Primary cells or a cell line derived from an animal according to any one of claims 1-12, wherein said primary cells are preferably mouse embryonic feeder cells (MEFs).

14. Use of the animal, primary cells, or cell lines according to any one of claims 1-13 as a model for:
(a) studying the molecular mechanisms of, or physiological processes associated with uncontrolled cell growth, such as cancer and/or metastasis formation, preferably in breast cancer, lung cancer, colorectal cancer, hepatocellular cancer, prostate cancer, melanoma, and/or pancreatic cancer;
(b) identification and/or testing of an agent useful in the prevention, amelioration or treatment of uncontrolled cell growth, such as cancer and/ or metastasis formation, preferably in breast cancer, lung cancer, colorectal cancer, hepatocellular cancer, prostate cancer, melanoma, and/or pancreatic cancer;
(c) identification of a protein and/or nucleic diagnostic marker for uncontrolled cell growth, such as cancer and/or metastasis formation, preferably in breast cancer, lung cancer, colorectal cancer, hepatocellular cancer, prostate cancer, melanoma, and/or pancreatic cancer; and/or
(d) studying the molecular mechanisms of, or physiological processes or medical conditions associated with undesirable activity, expression, or production of said modified FGFR4.

15. An modified FGFR4 polypeptide according to SEQ ID NO: 5 or SEQ ID NO: 6.

16. A nucleic acid molecule encoding the polypeptide of claim 15.

17. A rodent animal comprising an endogenous gene encoding a modified FGFR4 protein, wherein the modification is at least one amino acid substitution compared to the wild-type FGFR4 protein, preferably an FGFR4 protein according to SEQ ID NO: 1, which modification, if present in at least some or all or essentially all cells of said animal in a heterozygous or homozygous manner, results in a phenotype associated with an increased rate of tumor growth and/or metastasis formation compared to a wild-type animal.

18. A rodent animal according to claim 17 which additionally expresses in the genome of at least some of its cells a transgene encoding a TGF-α protein.

19. The animal of claim 18 wherein said transgene is expressed in mammary cells, preferably by expression under the control of the WAP-promotor.

20. The animal according to any one of claims 17-19, wherein said tumor is a mammary tumor.


SEQUENCE LISTING

<110> Max-Planck-Gesellschaft zur Förderung der Wissenschaften
e.V. -Max-Planck-Institut für Biochemie

<120> FGFR4 Arg388 Cancer Model

<130> 43589P EP

<141> 2008-09-24

<160> 14

<170> PatentIn version 3.3

<210> 1
<211> 799
<212> PRT
<213> Mus sp.

<220>
<221> MISC_FEATURE
<223> FGFR4

<400> 1

Met Trp Leu Leu Leu Ala Leu Leu Ser Ile Phe Gln Gly Thr Pro Ala
1               5                   10                  15

Leu Ser Leu Glu Ala Ser Glu Glu Met Glu Gln Glu Pro Cys Leu Ala
            20                  25                  30

Pro Ile Leu Glu Gln Gln Glu Gln Val Leu Thr Val Ala Leu Gly Gln
        35                  40                  45

Pro Val Arg Leu Cys Cys Gly Arg Thr Glu Arg Gly Arg His Trp Tyr
    50                  55                  60

Lys Glu Gly Ser Arg Leu Ala Ser Ala Gly Arg Val Arg Gly Trp Arg
65                  70                  75                  80

Gly Arg Leu Glu Ile Ala Ser Phe Leu Pro Glu Asp Ala Gly Arg Tyr
                85                  90                  95

Leu Cys Leu Ala Arg Gly Ser Met Thr Val Val His Asn Leu Thr Leu
            100                 105                 110

Leu Met Asp Asp Ser Leu Thr Ser Ile Ser Asn Asp Glu Asp Pro Lys
        115                 120                 125

Thr Leu Ser Ser Ser Ser Ser Gly His Val Tyr Pro Gln Gln Ala Pro
    130                 135                 140

Tyr Trp Thr His Pro Gln Arg Met Glu Lys Lys Leu His Ala Val Pro
145                 150                 155                 160

Ala Gly Asn Thr Val Lys Phe Arg Cys Pro Ala Ala Gly Asn Pro Met
                165                 170                 175

Pro Thr Ile His Trp Leu Lys Asp Gly Gln Ala Phe His Gly Glu Asn
            180                 185                 190

Arg Ile Gly Gly Ile Arg Leu Arg His Gln His Trp Ser Leu Val Met
        195                 200                 205

Glu Ser Val Val Pro Ser Asp Arg Gly Thr Tyr Thr Cys Leu Val Glu
    210                 215                 220

Asn Ser Leu Gly Ser Ile Arg Tyr Ser Tyr Leu Leu Asp Val Leu Glu
225                 230                 235                 240

Arg Ser Pro His Arg Pro Ile Leu Gln Ala Gly Leu Pro Ala Asn Thr
                245                 250                 255

Thr Ala Val Val Gly Ser Asp Val Glu Leu Leu Cys Lys Val Tyr Ser
            260                 265                 270

Asp Ala Gln Pro His Ile Gln Trp Leu Lys His Val Val Ile Asn Gly
        275                 280                 285

Ser Ser Phe Gly Ala Asp Gly Phe Pro Tyr Val Gln Val Leu Lys Thr
    290                 295                 300

Thr Asp Ile Asn Ser Ser Glu Val Glu Val Leu Tyr Leu Arg Asn Val
305                 310                 315                 320

Ser Ala Glu Asp Ala Gly Glu Tyr Thr Cys Leu Ala Gly Asn Ser Ile
                325                 330                 335

Gly Leu Ser Tyr Gln Ser Ala Trp Leu Thr Val Leu Pro Glu Glu Asp
            340                 345                 350

Leu Thr Trp Thr Thr Ala Thr Pro Glu Ala Arg Tyr Thr Asp Ile Ile
        355                 360                 365

Leu Tyr Val Ser Gly Ser Leu Val Leu Leu Val Leu Leu Leu Leu Ala
    370                 375                 380

Gly Val Tyr His Arg Gln Val Ile Arg Gly His Tyr Ser Arg Gln Pro
385                 390                 395                 400

Val Thr Ile Gln Lys Leu Ser Arg Phe Pro Leu Ala Arg Gln Phe Ser
                405                 410                 415

Leu Glu Ser Arg Ser Ser Gly Lys Ser Ser Leu Ser Leu Val Arg Gly
            420                 425                 430

Val Arg Leu Ser Ser Ser Gly Pro Pro Leu Leu Thr Gly Leu Val Asn
        435                 440                 445

Leu Asp Leu Pro Leu Asp Pro Leu Trp Glu Phe Pro Arg Asp Arg Leu
    450                 455                 460

Val Leu Gly Lys Pro Leu Gly Glu Gly Cys Phe Gly Gln Val Val Arg
465                 470                 475                 480

Ala Glu Ala Phe Gly Met Asp Pro Ser Arg Pro Asp Gln Thr Ser Thr
                485                 490                 495

Val Ala Val Lys Met Leu Lys Asp Asn Ala Ser Asp Lys Asp Leu Ala
            500                 505                 510

Asp Leu Val Ser Glu Met Glu Val Met Lys Leu Ile Gly Arg His Lys
        515                 520                 525

Asn Ile Ile Asn Leu Leu Gly Val Cys Thr Gln Glu Gly Pro Leu Tyr
    530                 535                 540

Val Ile Val Glu Cys Ala Ala Lys Gly Asn Leu Arg Glu Phe Leu Arg
545                 550                 555                 560

Ala Arg Arg Pro Pro Gly Pro Asp Leu Ser Pro Asp Gly Pro Arg Ser
                565                 570                 575

Ser Glu Gly Pro Leu Ser Phe Pro Ala Leu Val Ser Cys Ala Tyr Gln
            580                 585                 590

Val Ala Arg Gly Met Gln Tyr Leu Glu Ser Arg Lys Cys Ile His Arg
        595                 600                 605

Asp Leu Ala Ala Arg Asn Val Leu Val Thr Glu Asp Asp Val Met Lys
    610                 615                 620

Ile Ala Asp Phe Gly Leu Ala Arg Gly Val His His Ile Asp Tyr Tyr
625                 630                 635                 640

Lys Lys Thr Ser Asn Gly Arg Leu Pro Val Lys Trp Met Ala Pro Glu
                645                 650                 655

Ala Leu Phe Asp Arg Val Tyr Thr His Gln Ser Asp Val Trp Ser Phe
            660                 665                 670

Gly Ile Leu Leu Trp Glu Ile Phe Thr Leu Gly Gly Ser Pro Tyr Pro
        675                 680                 685

Gly Ile Pro Val Glu Glu Leu Phe Ser Leu Leu Arg Glu Gly His Arg
    690                 695                 700

Met Glu Arg Pro Pro Asn Cys Pro Ser Glu Leu Tyr Gly Leu Met Arg
705                 710                 715                 720

Glu Cys Trp His Ala Ala Pro Ser Gln Arg Pro Thr Phe Lys Gln Leu
                725                 730                 735

Val Glu Ala Leu Asp Lys Val Leu Leu Ala Val Ser Glu Glu Tyr Leu
            740                 745                 750

Asp Leu Arg Leu Thr Phe Gly Pro Phe Ser Pro Ser Asn Gly Asp Ala
        755                 760                 765

Ser Ser Thr Cys Ser Ser Ser Asp Ser Val Phe Ser His Asp Pro Leu
    770                 775                 780

Pro Leu Glu Pro Ser Pro Phe Pro Phe Ser Asp Ser Gln Thr Thr
785                 790                 795

<210>  2
<211>  802
<212>  PRT
<213>  Homo sapiens

<220>
<221>  MISC_FEATURE
<223>  isoform 1

<220>
<221>  MISC_FEATURE
<223>  FGFR4 human isoform 1

<400>  2

Met Arg Leu Leu Leu Ala Leu Leu Gly Val Leu Leu Ser Val Pro Gly
1               5                   10                  15

Pro Pro Val Leu Ser Leu Glu Ala Ser Glu Glu Val Glu Leu Glu Pro
            20                  25                  30

Cys Leu Ala Pro Ser Leu Glu Gln Gln Glu Gln Glu Leu Thr Val Ala
        35                  40                  45

Leu Gly Gln Pro Val Arg Leu Cys Cys Gly Arg Ala Glu Arg Gly Gly
    50                  55                  60

His Trp Tyr Lys Glu Gly Ser Arg Leu Ala Pro Ala Gly Arg Val Arg
65                  70                  75                  80

Gly Trp Arg Gly Arg Leu Glu Ile Ala Ser Phe Leu Pro Glu Asp Ala
                85                  90                  95

Gly Arg Tyr Leu Cys Leu Ala Arg Gly Ser Met Ile Val Leu Gln Asn
            100                 105                 110

Leu Thr Leu Ile Thr Gly Asp Ser Leu Thr Ser Ser Asn Asp Asp Glu
        115                 120                 125

Asp Pro Lys Ser His Arg Asp Pro Ser Asn Arg His Ser Tyr Pro Gln

130                 135                 140

Gln Ala Pro Tyr Trp Thr His Pro Gln Arg Met Glu Lys Lys Leu His
145                 150                 155                 160

Ala Val Pro Ala Gly Asn Thr Val Lys Phe Arg Cys Pro Ala Ala Gly
                165                 170                 175

Asn Pro Thr Pro Thr Ile Arg Trp Leu Lys Asp Gly Gln Ala Phe His
            180                 185                 190

Gly Glu Asn Arg Ile Gly Gly Ile Arg Leu Arg His Gln His Trp Ser
        195                 200                 205

Leu Val Met Glu Ser Val Val Pro Ser Asp Arg Gly Thr Tyr Thr Cys
    210                 215                 220

Leu Val Glu Asn Ala Val Gly Ser Ile Arg Tyr Asn Tyr Leu Leu Asp
225                 230                 235                 240

Val Leu Glu Arg Ser Pro His Arg Pro Ile Leu Gln Ala Gly Leu Pro
                245                 250                 255

Ala Asn Thr Thr Ala Val Val Gly Ser Asp Val Glu Leu Leu Cys Lys
            260                 265                 270

Val Tyr Ser Asp Ala Gln Pro His Ile Gln Trp Leu Lys His Ile Val
        275                 280                 285

Ile Asn Gly Ser Ser Phe Gly Ala Asp Gly Phe Pro Tyr Val Gln Val
    290                 295                 300

Leu Lys Thr Ala Asp Ile Asn Ser Ser Glu Val Glu Val Leu Tyr Leu
305                 310                 315                 320

Arg Asn Val Ser Ala Glu Asp Ala Gly Glu Tyr Thr Cys Leu Ala Gly
                325                 330                 335

Asn Ser Ile Gly Leu Ser Tyr Gln Ser Ala Trp Leu Thr Val Leu Pro
            340                 345                 350

Glu Glu Asp Pro Thr Trp Thr Ala Ala Ala Pro Glu Ala Arg Tyr Thr
        355                 360                 365

Asp Ile Ile Leu Tyr Ala Ser Gly Ser Leu Ala Leu Ala Val Leu Leu
    370                 375                 380

Leu Leu Ala Gly Leu Tyr Arg Gly Gln Ala Leu His Gly Arg His Pro
385                 390                 395                 400

Arg Pro Pro Ala Thr Val Gln Lys Leu Ser Arg Phe Pro Leu Ala Arg
                405                 410                 415

Gln Phe Ser Leu Glu Ser Gly Ser Ser Gly Lys Ser Ser Ser Ser Leu
            420                 425                 430

Val Arg Gly Val Arg Leu Ser Ser Ser Gly Pro Ala Leu Leu Ala Gly
        435                 440                 445

Leu Val Ser Leu Asp Leu Pro Leu Asp Pro Leu Trp Glu Phe Pro Arg
    450                 455                 460

Asp Arg Leu Val Leu Gly Lys Pro Leu Gly Glu Gly Cys Phe Gly Gln
465                 470                 475                 480

Val Val Arg Ala Glu Ala Phe Gly Met Asp Pro Ala Arg Pro Asp Gln
                485                 490                 495

Ala Ser Thr Val Ala Val Lys Met Leu Lys Asp Asn Ala Ser Asp Lys
            500                 505                 510

Asp Leu Ala Asp Leu Val Ser Glu Met Glu Val Met Lys Leu Ile Gly
        515                 520                 525

Arg His Lys Asn Ile Ile Asn Leu Leu Gly Val Cys Thr Gln Glu Gly
    530                 535                 540

Pro Leu Tyr Val Ile Val Glu Cys Ala Ala Lys Gly Asn Leu Arg Glu
545                 550                 555                 560

Phe Leu Arg Ala Arg Arg Pro Pro Gly Pro Asp Leu Ser Pro Asp Gly
                565                 570                 575

Pro Arg Ser Ser Glu Gly Pro Leu Ser Phe Pro Val Leu Val Ser Cys
            580                 585                 590

Ala Tyr Gln Val Ala Arg Gly Met Gln Tyr Leu Glu Ser Arg Lys Cys
        595                 600                 605

Ile His Arg Asp Leu Ala Ala Arg Asn Val Leu Val Thr Glu Asp Asn
    610                 615                 620

Val Met Lys Ile Ala Asp Phe Gly Leu Ala Arg Gly Val His His Ile
625                 630                 635                 640

Asp Tyr Tyr Lys Lys Thr Ser Asn Gly Arg Leu Pro Val Lys Trp Met
                645                 650                 655

Ala Pro Glu Ala Leu Phe Asp Arg Val Tyr Thr His Gln Ser Asp Val
            660                 665                 670

Trp Ser Phe Gly Ile Leu Leu Trp Glu Ile Phe Thr Leu Gly Gly Ser
        675                 680                 685

Pro Tyr Pro Gly Ile Pro Val Glu Glu Leu Phe Ser Leu Leu Arg Glu
    690                 695                 700

Gly His Arg Met Asp Arg Pro Pro His Cys Pro Pro Glu Leu Tyr Gly
705                 710                 715                 720

Leu Met Arg Glu Cys Trp His Ala Ala Pro Ser Gln Arg Pro Thr Phe
                725                 730                 735

Lys Gln Leu Val Glu Ala Leu Asp Lys Val Leu Leu Ala Val Ser Glu
            740                 745                 750

Glu Tyr Leu Asp Leu Arg Leu Thr Phe Gly Pro Tyr Ser Pro Ser Gly
        755                 760                 765

Gly Asp Ala Ser Ser Thr Cys Ser Ser Ser Asp Ser Val Phe Ser His
    770                 775                 780

Asp Pro Leu Pro Leu Gly Ser Ser Ser Phe Pro Phe Gly Ser Gly Val
785                 790                 795                 800

Gln Thr

<210>  3
<211>  802
<212>  PRT
<213>  Homo sapiens

<220>
<221>  MISC_FEATURE
<223>  FGFR4 human isoform 2

<400>  3

Met Arg Leu Leu Leu Ala Leu Leu Gly Val Leu Leu Ser Val Pro Gly
1               5                   10                  15

Pro Pro Val Leu Ser Leu Glu Ala Ser Glu Glu Val Glu Leu Glu Pro
            20                  25                  30

Cys Leu Ala Pro Ser Leu Glu Gln Gln Glu Gln Glu Leu Thr Val Ala
        35                  40                  45

Leu Gly Gln Pro Val Arg Leu Cys Cys Gly Arg Ala Glu Arg Gly Gly
    50                  55                  60

His Trp Tyr Lys Glu Gly Ser Arg Leu Ala Pro Ala Gly Arg Val Arg
65                  70                  75                  80

Gly Trp Arg Gly Arg Leu Glu Ile Ala Ser Phe Leu Pro Glu Asp Ala
                85                  90                  95

Gly Arg Tyr Leu Cys Leu Ala Arg Gly Ser Met Ile Val Leu Gln Asn
            100                 105                 110

Leu Thr Leu Ile Thr Gly Asp Ser Leu Thr Ser Ser Asn Asp Asp Glu
        115                 120                 125

Asp Pro Lys Ser His Arg Asp Pro Ser Asn Arg His Ser Tyr Pro Gln
    130                 135                 140

Gln Ala Pro Tyr Trp Thr His Pro Gln Arg Met Glu Lys Lys Leu His
145                 150                 155                 160

Ala Val Pro Ala Gly Asn Thr Val Lys Phe Arg Cys Pro Ala Ala Gly
                165                 170                 175

Asn Pro Thr Pro Thr Ile Arg Trp Leu Lys Asp Gly Gln Ala Phe His
            180                 185                 190

Gly Glu Asn Arg Ile Gly Gly Ile Arg Leu Arg His Gln His Trp Ser
        195                 200                 205

Leu Val Met Glu Ser Val Val Pro Ser Asp Arg Gly Thr Tyr Thr Cys
    210                 215                 220

Leu Val Glu Asn Ala Val Gly Ser Ile Arg Tyr Asn Tyr Leu Leu Asp
225                 230                 235                 240

Val Leu Glu Arg Ser Pro His Arg Pro Ile Leu Gln Ala Gly Leu Pro
                245                 250                 255

Ala Asn Thr Thr Ala Val Val Gly Ser Asp Val Glu Leu Leu Cys Lys
            260                 265                 270

Val Tyr Ser Asp Ala Gln Pro His Ile Gln Trp Leu Lys His Ile Val
        275                 280                 285

Ile Asn Gly Ser Ser Phe Gly Ala Asp Gly Phe Pro Tyr Val Gln Val
    290                 295                 300

Leu Lys Thr Ala Asp Ile Asn Ser Ser Glu Val Glu Val Leu Tyr Leu
305                 310                 315                 320

Arg Asn Val Ser Ala Glu Asp Ala Gly Glu Tyr Thr Cys Leu Ala Gly
                325                 330                 335

Asn Ser Ile Gly Leu Ser Tyr Gln Ser Ala Trp Leu Thr Val Leu Pro
            340                 345                 350

Glu Glu Asp Pro Thr Trp Thr Ala Ala Ala Pro Glu Ala Arg Tyr Thr
        355                 360                 365

Asp Ile Ile Leu Tyr Ala Ser Gly Ser Leu Ala Leu Ala Val Leu Leu

370                 375                 380

Leu Leu Ala Gly Leu Tyr Arg Gly Gln Ala Leu His Gly Arg His Pro
385                 390                 395                 400

Arg Pro Pro Ala Thr Val Gln Lys Leu Ser Arg Phe Pro Leu Ala Arg
                405                 410                 415

Gln Phe Ser Leu Glu Ser Gly Ser Ser Gly Lys Ser Ser Ser Ser Leu
            420                 425                 430

Val Arg Gly Val Arg Leu Ser Ser Ser Gly Pro Ala Leu Leu Ala Gly
        435                 440                 445

Leu Val Ser Leu Asp Leu Pro Leu Asp Pro Leu Trp Glu Phe Pro Arg
    450                 455                 460

Asp Arg Leu Val Leu Gly Lys Pro Leu Gly Glu Gly Cys Phe Gly Gln
465                 470                 475                 480

Val Val Arg Ala Glu Ala Phe Gly Met Asp Pro Ala Arg Pro Asp Gln
                485                 490                 495

Ala Ser Thr Val Ala Val Lys Met Leu Lys Asp Asn Ala Ser Asp Lys
            500                 505                 510

Asp Leu Ala Asp Leu Val Ser Glu Met Glu Val Met Lys Leu Ile Gly
        515                 520                 525

Arg His Lys Asn Ile Ile Asn Leu Leu Gly Val Cys Thr Gln Glu Gly
    530                 535                 540

Pro Leu Tyr Val Ile Val Glu Cys Ala Ala Lys Gly Asn Leu Arg Glu
545                 550                 555                 560

Phe Leu Arg Ala Arg Arg Pro Pro Gly Pro Asp Leu Ser Pro Asp Gly
                565                 570                 575

Pro Arg Ser Ser Glu Gly Pro Leu Ser Phe Pro Val Leu Val Ser Cys
            580                 585                 590

Ala Tyr Gln Val Ala Arg Gly Met Gln Tyr Leu Glu Ser Arg Lys Cys
        595                 600                 605

Ile His Arg Asp Leu Ala Ala Arg Asn Val Leu Val Thr Glu Asp Asn
    610                 615                 620

Val Met Lys Ile Ala Asp Phe Gly Leu Ala Arg Gly Val His His Ile
625                 630                 635                 640

Asp Tyr Tyr Lys Lys Thr Ser Asn Gly Arg Leu Pro Val Lys Trp Met
                645                 650                 655

Ala Pro Glu Ala Leu Phe Asp Arg Val Tyr Thr His Gln Ser Asp Val
            660                 665                 670

Trp Ser Phe Gly Ile Leu Leu Trp Glu Ile Phe Thr Leu Gly Gly Ser
        675                 680                 685

Pro Tyr Pro Gly Ile Pro Val Glu Glu Leu Phe Ser Leu Leu Arg Glu
    690                 695                 700

Gly His Arg Met Asp Arg Pro Pro His Cys Pro Pro Glu Leu Tyr Gly
705                 710                 715                 720

Leu Met Arg Glu Cys Trp His Ala Ala Pro Ser Gln Arg Pro Thr Phe
                725                 730                 735

Lys Gln Leu Val Glu Ala Leu Asp Lys Val Leu Leu Ala Val Ser Glu
            740                 745                 750

Glu Tyr Leu Asp Leu Arg Leu Thr Phe Gly Pro Tyr Ser Pro Ser Gly
        755                 760                 765

Gly Asp Ala Ser Ser Thr Cys Ser Ser Ser Asp Ser Val Phe Ser His
    770                 775                 780

Asp Pro Leu Pro Leu Gly Ser Ser Ser Phe Pro Phe Gly Ser Gly Val
785                 790                 795                 800

Gln Thr

<210>  4
<211>  800
<212>  PRT
<213>  Rattus norvegicus

<220>
<221>  MISC_FEATURE
<223>  FGFR4

<400>  4
   Met Trp Leu Leu Leu Ala Leu Leu Ser Ile Phe Gln Glu Thr Pro Ala
   1               5                   10                  15
   Phe Ser Leu Glu Ala Ser Glu Glu Met Glu Gln Glu Pro Cys Pro Ala
               20                  25                  30
   Pro Ile Ser Glu Gln Gln Glu Gln Val Leu Thr Val Ala Leu Gly Gln
           35                  40                  45
   Pro Val Arg Leu Cys Cys Gly Arg Thr Glu Arg Gly Arg His Trp Tyr
       50                  55                  60
   Lys Glu Gly Ser Arg Leu Ala Ser Ala Gly Arg Val Arg Gly Trp Arg
   65                  70                  75                  80
   Gly Arg Leu Glu Ile Ala Ser Phe Leu Pro Glu Asp Ala Gly Arg Tyr
                   85                  90                  95
   Leu Cys Leu Ala Arg Gly Ser Met Thr Val Val His Asn Leu Thr Leu
               100                 105                 110
   Ile Met Asp Asp Ser Leu Pro Ser Ile Asn Asn Glu Asp Pro Lys Thr
           115                 120                 125
   Leu Ser Ser Ser Ser Ser Gly His Ser Tyr Leu Gln Gln Ala Pro Tyr
       130                 135                 140
   Trp Thr His Pro Gln Arg Met Glu Lys Lys Leu His Ala Val Pro Ala
   145                 150                 155                 160
   Gly Asn Thr Val Lys Phe Arg Cys Pro Ala Ala Gly Asn Pro Met Pro
                   165                 170                 175
   Thr Ile His Trp Leu Lys Asn Gly Gln Ala Phe His Gly Glu Asn Arg
               180                 185                 190
   Ile Gly Gly Ile Arg Leu Arg His Gln His Trp Ser Leu Val Met Glu
           195                 200                 205
   Ser Val Val Pro Ser Asp Arg Gly Thr Tyr Thr Cys Leu Val Glu Asn
       210                 215                 220
   Ser Leu Gly Ser Ile Arg Tyr Ser Tyr Leu Leu Asp Val Leu Glu Arg
   225                 230                 235                 240
   Ser Pro His Arg Pro Ile Leu Gln Ala Gly Leu Pro Ala Asn Thr Thr
                   245                 250                 255
   Ala Val Val Gly Ser Asn Val Glu Leu Leu Cys Lys Val Tyr Ser Asp
               260                 265                 270
   Ala Gln Pro His Ile Gln Trp Leu Lys His Ile Val Ile Asn Gly Ser
           275                 280                 285
   Ser Phe Gly Ala Asp Gly Phe Pro Tyr Val Gln Val Leu Lys Thr Thr
       290                 295                 300
   Asp Ile Asn Ser Ser Glu Val Glu Val Leu Tyr Leu Arg Asn Val Ser
   305                 310                 315                 320
   Ala Glu Asp Ala Gly Glu Tyr Thr Cys Leu Ala Gly Asn Ser Ile Gly
                   325                 330                 335
   Leu Ser Tyr Gln Ser Ala Trp Leu Thr Val Leu Pro Ala Glu Glu Glu
               340                 345                 350
   Asp Leu Ala Trp Thr Thr Ala Thr Ser Glu Ala Arg Tyr Thr Asp Ile
           355                 360                 365
   Ile Leu Tyr Val Ser Gly Ser Leu Ala Leu Val Leu Leu Leu Leu Leu
       370                 375                 380
   Ala Gly Val Tyr His Arg Gln Ala Ile His Gly His His Ser Arg Gln
   385                 390                 395                 400
   Pro Val Thr Val Gln Lys Leu Ser Arg Phe Pro Leu Ala Arg Gln Phe
                   405                 410                 415
   Ser Leu Glu Ser Arg Ser Ser Gly Lys Ser Ser Leu Ser Leu Val Arg
               420                 425                 430
   Gly Val Arg Leu Ser Ser Ser Gly Pro Pro Leu Leu Thr Gly Leu Val
           435                 440                 445
   Ser Leu Asp Leu Pro Leu Asp Pro Leu Trp Glu Phe Pro Arg Asp Arg
       450                 455                 460
   Leu Val Leu Gly Lys Pro Leu Gly Glu Gly Cys Phe Gly Gln Val Val
   465                 470                 475                 480
   Arg Ala Glu Ala Leu Gly Met Asp Ser Ser Arg Pro Asp Gln Thr Ser
                   485                 490                 495
   Thr Val Ala Val Lys Met Leu Lys Asp Asn Ala Ser Asp Lys Asp Leu
               500                 505                 510
   Ala Asp Leu Ile Ser Glu Met Glu Met Met Lys Leu Ile Gly Arg His
           515                 520                 525
   Lys Asn Ile Ile Asn Leu Leu Gly Val Cys Thr Gln Glu Gly Pro Leu
       530                 535                 540
   Tyr Val Ile Val Glu Tyr Ala Ala Lys Gly Asn Leu Arg Glu Phe Leu
   545                 550                 555                 560
   Arg Ala Arg Arg Pro Pro Gly Pro Asp Leu Ser Pro Asp Gly Pro Arg
                   565                 570                 575
   Ser Ser Glu Gly Pro Leu Ser Phe Pro Ala Leu Val Ser Cys Ala Tyr
               580                 585                 590
   Gln Val Ala Arg Gly Met Gln Tyr Leu Glu Ser Arg Lys Cys Ile His
           595                 600                 605
   Arg Asp Leu Ala Ala Arg Asn Val Leu Val Thr Glu Asp Asp Val Met
   610                     615                     620
   Lys Ile Ala Asp Phe Gly Leu Ala Arg Gly Val His His Ile Asp Tyr
   625                 630                 635                 640
   Tyr Lys Lys Thr Ser Asn Gly Arg Leu Pro Val Lys Trp Met Ala Pro
                   645                 650                 655
   Glu Ala Leu Phe Asp Arg Val Tyr Thr His Gln Ser Asp Val Trp Ser
               660                 665                 670
   Phe Gly Ile Leu Leu Trp Glu Ile Phe Thr Leu Gly Gly Ser Pro Tyr
           675                 680                 685
   Pro Gly Ile Pro Val Glu Glu Leu Phe Ser Leu Leu Arg Glu Gly His
       690                 695                 700
   Arg Met Glu Arg Pro Pro Asn Cys Pro Ser Glu Leu Tyr Gly Leu Met
   705                 710                 715                 720
   Arg Glu Cys Trp His Ala Ala Pro Ser Gln Arg Pro Thr Phe Lys Gln
                   725                 730                 735
   Leu Val Glu Ala Leu Asp Lys Val Leu Leu Ala Val Ser Glu Glu Tyr
               740                 745                 750
   Leu Asp Leu Arg Leu Thr Phe Gly Pro Tyr Ser Pro Asn Asn Gly Asp
           755                 760                 765
   Ala Ser Ser Thr Cys Ser Ser Ser Asp Ser Val Phe Ser His Asp Pro
       770                 775                 780
   Leu Pro Leu Glu Pro Ser Pro Phe Pro Phe Pro Glu Ala Gln Thr Thr
   785                 790                 795                 800

<210>  5
<211>  799
<212>  PRT
<213>  Mus sp.

<220>
<221>  MISC_FEATURE
<223>  FGFR4: G to R substitution

<400>  5

Met Trp Leu Leu Leu Ala Leu Leu Ser Ile Phe Gln Gly Thr Pro Ala
1               5                   10                  15

Leu Ser Leu Glu Ala Ser Glu Glu Met Glu Gln Glu Pro Cys Leu Ala
            20                  25                  30

Pro Ile Leu Glu Gln Gln Glu Gln Val Leu Thr Val Ala Leu Gly Gln
        35                  40                  45

Pro Val Arg Leu Cys Cys Gly Arg Thr Glu Arg Gly Arg His Trp Tyr
    50                  55                  60

Lys Glu Gly Ser Arg Leu Ala Ser Ala Gly Arg Val Arg Gly Trp Arg
65                  70                  75                  80

Gly Arg Leu Glu Ile Ala Ser Phe Leu Pro Glu Asp Ala Gly Arg Tyr
                85                  90                  95

Leu Cys Leu Ala Arg Gly Ser Met Thr Val Val His Asn Leu Thr Leu
            100                 105                 110

Leu Met Asp Asp Ser Leu Thr Ser Ile Ser Asn Asp Glu Asp Pro Lys
        115                 120                 125

Thr Leu Ser Ser Ser Ser Ser Gly His Val Tyr Pro Gln Gln Ala Pro
    130                 135                 140

Tyr Trp Thr His Pro Gln Arg Met Glu Lys Lys Leu His Ala Val Pro
145                 150                 155                 160

Ala Gly Asn Thr Val Lys Phe Arg Cys Pro Ala Ala Gly Asn Pro Met
                165                 170                 175

Pro Thr Ile His Trp Leu Lys Asp Gly Gln Ala Phe His Gly Glu Asn
            180                 185                 190

Arg Ile Gly Gly Ile Arg Leu Arg His Gln His Trp Ser Leu Val Met
        195                 200                 205

Glu Ser Val Val Pro Ser Asp Arg Gly Thr Tyr Thr Cys Leu Val Glu
    210                 215                 220

Asn Ser Leu Gly Ser Ile Arg Tyr Ser Tyr Leu Leu Asp Val Leu Glu
225                 230                 235                 240

Arg Ser Pro His Arg Pro Ile Leu Gln Ala Gly Leu Pro Ala Asn Thr
                245                 250                 255

Thr Ala Val Val Gly Ser Asp Val Glu Leu Leu Cys Lys Val Tyr Ser
            260                 265                 270

Asp Ala Gln Pro His Ile Gln Trp Leu Lys His Val Val Ile Asn Gly
        275                 280                 285

Ser Ser Phe Gly Ala Asp Gly Phe Pro Tyr Val Gln Val Leu Lys Thr
    290                 295                 300

Thr Asp Ile Asn Ser Ser Glu Val Glu Val Leu Tyr Leu Arg Asn Val
305                 310                 315                 320

Ser Ala Glu Asp Ala Gly Glu Tyr Thr Cys Leu Ala Gly Asn Ser Ile
                325                 330                 335

Gly Leu Ser Tyr Gln Ser Ala Trp Leu Thr Val Leu Pro Glu Glu Asp
            340                 345                 350

Leu Thr Trp Thr Thr Ala Thr Pro Glu Ala Arg Tyr Thr Asp Ile Ile
        355                 360                 365

Leu Tyr Val Ser Gly Ser Leu Val Leu Leu Val Leu Leu Leu Leu Ala
    370                 375                 380

Arg Val Tyr His Arg Gln Val Ile Arg Gly His Tyr Ser Arg Gln Pro
385                 390                 395                 400

Val Thr Ile Gln Lys Leu Ser Arg Phe Pro Leu Ala Arg Gln Phe Ser
                405                 410                 415

Leu Glu Ser Arg Ser Ser Gly Lys Ser Ser Leu Ser Leu Val Arg Gly
            420                 425                 430

Val Arg Leu Ser Ser Ser Gly Pro Pro Leu Leu Thr Gly Leu Val Asn
        435                 440                 445

Leu Asp Leu Pro Leu Asp Pro Leu Trp Glu Phe Pro Arg Asp Arg Leu
    450                 455                 460

Val Leu Gly Lys Pro Leu Gly Glu Gly Cys Phe Gly Gln Val Val Arg
465                 470                 475                 480

Ala Glu Ala Phe Gly Met Asp Pro Ser Arg Pro Asp Gln Thr Ser Thr
                485                 490                 495

Val Ala Val Lys Met Leu Lys Asp Asn Ala Ser Asp Lys Asp Leu Ala
            500                 505                 510

Asp Leu Val Ser Glu Met Glu Val Met Lys Leu Ile Gly Arg His Lys
        515                 520                 525

Asn Ile Ile Asn Leu Leu Gly Val Cys Thr Gln Glu Gly Pro Leu Tyr
    530                 535                 540

Val Ile Val Glu Cys Ala Ala Lys Gly Asn Leu Arg Glu Phe Leu Arg
545                 550                 555                 560

Ala Arg Arg Pro Pro Gly Pro Asp Leu Ser Pro Asp Gly Pro Arg Ser
                565                 570                 575

Ser Glu Gly Pro Leu Ser Phe Pro Ala Leu Val Ser Cys Ala Tyr Gln
            580                 585                 590

Val Ala Arg Gly Met Gln Tyr Leu Glu Ser Arg Lys Cys Ile His Arg
        595                 600                 605

Asp Leu Ala Ala Arg Asn Val Leu Val Thr Glu Asp Asp Val Met Lys
    610                 615                 620

Ile Ala Asp Phe Gly Leu Ala Arg Gly Val His His Ile Asp Tyr Tyr
625                 630                 635                 640

Lys Lys Thr Ser Asn Gly Arg Leu Pro Val Lys Trp Met Ala Pro Glu
                645                 650                 655

Ala Leu Phe Asp Arg Val Tyr Thr His Gln Ser Asp Val Trp Ser Phe
            660                 665                 670

Gly Ile Leu Leu Trp Glu Ile Phe Thr Leu Gly Gly Ser Pro Tyr Pro
        675                 680                 685

Gly Ile Pro Val Glu Glu Leu Phe Ser Leu Leu Arg Glu Gly His Arg
    690                 695                 700

Met Glu Arg Pro Pro Asn Cys Pro Ser Glu Leu Tyr Gly Leu Met Arg
705                 710                 715                 720

Glu Cys Trp His Ala Ala Pro Ser Gln Arg Pro Thr Phe Lys Gln Leu
                725                 730                 735

Val Glu Ala Leu Asp Lys Val Leu Leu Ala Val Ser Glu Glu Tyr Leu
            740                 745                 750

Asp Leu Arg Leu Thr Phe Gly Pro Phe Ser Pro Ser Asn Gly Asp Ala
        755                 760                 765

Ser Ser Thr Cys Ser Ser Ser Asp Ser Val Phe Ser His Asp Pro Leu
    770                 775                 780

Pro Leu Glu Pro Ser Pro Phe Pro Phe Ser Asp Ser Gln Thr Thr
785                 790                 795

<210>  6
<211>  800
<212>  PRT
<213>  Rattus norvegicus

<220>
<221>  MISC_FEATURE
<223>  FGFR4: G to R substitution

<400>  6

Met Trp Leu Leu Leu Ala Leu Leu Ser Ile Phe Gln Glu Thr Pro Ala
1               5                   10                  15

Phe Ser Leu Glu Ala Ser Glu Glu Met Glu Gln Glu Pro Cys Pro Ala

20                          25                          30

Pro Ile Ser Glu Gln Gln Glu Gln Val Leu Thr Val Ala Leu Gly Gln
        35                          40                          45

Pro Val Arg Leu Cys Cys Gly Arg Thr Glu Arg Gly Arg His Trp Tyr
    50                          55                          60

Lys Glu Gly Ser Arg Leu Ala Ser Ala Gly Arg Val Arg Gly Trp Arg
65                          70                          75                          80

Gly Arg Leu Glu Ile Ala Ser Phe Leu Pro Glu Asp Ala Gly Arg Tyr
                    85                          90                          95

Leu Cys Leu Ala Arg Gly Ser Met Thr Val Val His Asn Leu Thr Leu
                100                         105                         110

Ile Met Asp Asp Ser Leu Pro Ser Ile Asn Asn Glu Asp Pro Lys Thr
        115                         120                         125

Leu Ser Ser Ser Ser Ser Gly His Ser Tyr Leu Gln Gln Ala Pro Tyr
    130                         135                         140

Trp Thr His Pro Gln Arg Met Glu Lys Lys Leu His Ala Val Pro Ala
145                         150                         155                         160

Gly Asn Thr Val Lys Phe Arg Cys Pro Ala Ala Gly Asn Pro Met Pro
                    165                         170                         175

Thr Ile His Trp Leu Lys Asn Gly Gln Ala Phe His Gly Glu Asn Arg
                180                         185                         190

Ile Gly Gly Ile Arg Leu Arg His Gln His Trp Ser Leu Val Met Glu
        195                         200                         205

Ser Val Val Pro Ser Asp Arg Gly Thr Tyr Thr Cys Leu Val Glu Asn
    210                         215                         220

Ser Leu Gly Ser Ile Arg Tyr Ser Tyr Leu Leu Asp Val Leu Glu Arg
225                         230                         235                         240

Ser Pro His Arg Pro Ile Leu Gln Ala Gly Leu Pro Ala Asn Thr Thr
                    245                         250                         255

Ala Val Val Gly Ser Asn Val Glu Leu Leu Cys Lys Val Tyr Ser Asp
                260                         265                         270

Ala Gln Pro His Ile Gln Trp Leu Lys His Ile Val Ile Asn Gly Ser
        275                         280                         285

Ser Phe Gly Ala Asp Gly Phe Pro Tyr Val Gln Val Leu Lys Thr Thr
    290                         295                         300

Asp Ile Asn Ser Ser Glu Val Glu Val Leu Tyr Leu Arg Asn Val Ser
305                 310                 315                 320

Ala Glu Asp Ala Gly Glu Tyr Thr Cys Leu Ala Gly Asn Ser Ile Gly
                325                 330                 335

Leu Ser Tyr Gln Ser Ala Trp Leu Thr Val Leu Pro Ala Glu Glu Glu
            340                 345                 350

Asp Leu Ala Trp Thr Thr Ala Thr Ser Glu Ala Arg Tyr Thr Asp Ile
        355                 360                 365

Ile Leu Tyr Val Ser Gly Ser Leu Ala Leu Val Leu Leu Leu Leu Leu
    370                 375                 380

Ala Arg Val Tyr His Arg Gln Ala Ile His Gly His His Ser Arg Gln
385                 390                 395                 400

Pro Val Thr Val Gln Lys Leu Ser Arg Phe Pro Leu Ala Arg Gln Phe
                405                 410                 415

Ser Leu Glu Ser Arg Ser Ser Gly Lys Ser Ser Leu Ser Leu Val Arg
            420                 425                 430

Gly Val Arg Leu Ser Ser Ser Gly Pro Pro Leu Leu Thr Gly Leu Val
        435                 440                 445

Ser Leu Asp Leu Pro Leu Asp Pro Leu Trp Glu Phe Pro Arg Asp Arg
    450                 455                 460

Leu Val Leu Gly Lys Pro Leu Gly Glu Gly Cys Phe Gly Gln Val Val
465                 470                 475                 480

Arg Ala Glu Ala Leu Gly Met Asp Ser Ser Arg Pro Asp Gln Thr Ser
                485                 490                 495

Thr Val Ala Val Lys Met Leu Lys Asp Asn Ala Ser Asp Lys Asp Leu
            500                 505                 510

Ala Asp Leu Ile Ser Glu Met Glu Met Met Lys Leu Ile Gly Arg His
        515                 520                 525

Lys Asn Ile Ile Asn Leu Leu Gly Val Cys Thr Gln Glu Gly Pro Leu
    530                 535                 540

Tyr Val Ile Val Glu Tyr Ala Ala Lys Gly Asn Leu Arg Glu Phe Leu
545                 550                 555                 560

Arg Ala Arg Arg Pro Pro Gly Pro Asp Leu Ser Pro Asp Gly Pro Arg
                565                 570                 575

Ser Ser Glu Gly Pro Leu Ser Phe Pro Ala Leu Val Ser Cys Ala Tyr
            580                 585                 590

Gln Val Ala Arg Gly Met Gln Tyr Leu Glu Ser Arg Lys Cys Ile His
        595                 600                 605

Arg Asp Leu Ala Ala Arg Asn Val Leu Val Thr Glu Asp Asp Val Met
    610                 615                 620

Lys Ile Ala Asp Phe Gly Leu Ala Arg Gly Val His His Ile Asp Tyr
625                 630                 635                 640

Tyr Lys Lys Thr Ser Asn Gly Arg Leu Pro Val Lys Trp Met Ala Pro
                645                 650                 655

Glu Ala Leu Phe Asp Arg Val Tyr Thr His Gln Ser Asp Val Trp Ser
            660                 665                 670

Phe Gly Ile Leu Leu Trp Glu Ile Phe Thr Leu Gly Gly Ser Pro Tyr
        675                 680                 685

Pro Gly Ile Pro Val Glu Glu Leu Phe Ser Leu Leu Arg Glu Gly His
    690                 695                 700

Arg Met Glu Arg Pro Pro Asn Cys Pro Ser Glu Leu Tyr Gly Leu Met
705                 710                 715                 720

Arg Glu Cys Trp His Ala Ala Pro Ser Gln Arg Pro Thr Phe Lys Gln
                725                 730                 735

Leu Val Glu Ala Leu Asp Lys Val Leu Leu Ala Val Ser Glu Glu Tyr
            740                 745                 750

Leu Asp Leu Arg Leu Thr Phe Gly Pro Tyr Ser Pro Asn Asn Gly Asp
        755                 760                 765

Ala Ser Ser Thr Cys Ser Ser Ser Asp Ser Val Phe Ser His Asp Pro
    770                 775                 780

Leu Pro Leu Glu Pro Ser Pro Phe Pro Phe Pro Glu Ala Gln Thr Thr
785                 790                 795                 800

<210>  7
<211>  30
<212>  DNA
<213>  Artificial

<220>
<223>  neoR-specific primer

<400>  7
aggatctcct gtcatctcac cttcctcctg                                        30

<210>  8
<211>  30
<212>  DNA
<213>  Artificial

<220>
<223>  neoR-specific primer

<400>  8
aagaactcgt caagaaggcg atagaaggcg                                        30


<210>  9
<211>  18
<212>  DNA
<213>  Artificial

<220>
<223>  Cre-specific primer

<400>  9
aacatgcttc atcgtcgg                                                     18


<210>  10
<211>  20
<212>  DNA
<213>  Artificial

<220>
<223>  Cre-specific primer

<400>  10
ttaggatcat cagctacacc                                                   20


<210>  11
<211>  21
<212>  DNA
<213>  Artificial

<220>
<223>  FGFR4-primer forward

<400>  11
cgtggacaac agcaacccct g                                                 21


<210>  12
<211>  22
<212>  DNA
<213>  Artificial

<220>
<223>  FGFR4-primer reverse

<400>  12
gctggcgaga gtagtggcca cg                                                22


<210>  13
<211>  21
<212>  DNA
<213>  Artificial

<220>
<223>  TGF-alpha primer forward

<400>  13

tgtcaggctc tggagaacag c                                                    21

<210>  14
<211>  21
<212>  DNA
<213>  Artificial

<220>
<223>  TGF-alpha primer reverse

<400>  14
cacagcgaac acccacgtac c                                                    21